# EUROPEAN PATENT SPECIFICATION

(11) **EP 3 979 982 B1**
(45) Date of publication and mention of the grant of the patent: **05.04.2023**
(21) Application number: 20736427.4
(22) Date of filing: 05.06.2020
(51) Int. Cl.: A61K 8/99, A61K 8/73, A61Q 19/08, A61K 31/728, A61K 35/747, A61P 17/16, A61P 17/18, A61K 9/06

(54) **COMPOSITIONS COMPRISING A BACTERIAL STRAIN LACTOBACILLUS PARACASEI AND HYALURONIC ACID AND THE USE THEREOF FOR THE TREATMENT OF THE SKIN**
ZUSAMMENSETZUNGEN MIT BAKTERIENSTAMM LACTOBACILLUS PARACASEI UND HYALURONSÄURE UND DEREN VERWENDUNG ZUR BEHANDLUNG DER HAUT
COMPOSITIONS COMPRENANT UNE SOUCHE BACTÉRIENNE LACTOBACILLUS PARACASEI ET DE L'ACIDE HYALURONIQUE ET LEUR UTILISATION DANS LE TRAITEMENT DE LA PEAU

(30) Priority: 05.06.2019 IT 201900008097
(43) Date of publication of application: 13.04.2022
(73) Proprietor: LAC2BIOME S.R.L., 20154 Milano (IT)
(72) Inventor: BIFFI, Andrea, 20060 Trezzano Rosa [MI] (IT)
(74) Representative: Benedetto, Marco
(86) International application number: PCT/IB2020/055326
(87) International publication number: WO 2020/245797

(56) References cited:
- WO-A1-2018/216744
- WO-A1-2019/111189
- CN-A- 106 902 073
- KR-A- 20140 127 437
- KR-A- 20160 092 569
- DATABASE GNPD [Online] MINTEL; 31 January 2019 (2019-01-31), anonymous: "Molecular Multi-Nutrient Day Cream", XP055668681, retrieved from www.gnpd.com Database accession no. 6312455
- DATABASE GNPD [Online] MINTEL; 25 July 2018 (2018-07-25), anonymous: "Probiotic Mask 2X", XP055628089, retrieved from www.gnpd.com Database accession no. 5824175

## Description

The present invention relates to a composition comprising probiotics (live and viable or inactivated bacterial strains) and hyaluronic acid or a salt thereof for use in the prevention, in the treatment (therapeutic or cosmetic curative) or in the attenuation of at least one sign or symptom associated with, or caused by, an skin aging or by a reduction in the immune defences of the skin or by an inflammation/infection of the skin, such as an inflammation induced or caused by UV rays.

### STATE OF THE ART

The skin is the outermost lining of the human body and it constitutes the first line of defence of the body against external aggressions, given that it acts as an anatomical barrier against potential pathogens and possible harmful agents. Skin changes may facilitate the onset of skin diseases, particularly caused by pathogens. For example, as the skin ages, the skin becomes thinner and more fragile, this being due to the fact that cell regeneration becomes slower and goes from normal 3-4 weeks to 4 or even 6 weeks. The skin undergoes structural changes with the passage of time, caused by several factors of different origin, which determine the loss of skin hydration, the onset of micro-wrinkles, the loss of elasticity, hyperkeratosis and the formation of hyperpigmented spots. Furthermore, the rich microbiome that colonises the human skin, performing important and useful functions in combating the adhesion and the development of skin pathogens, such as by way of example *Staphylococcus aureus, Pseudomonas aeruginosa, Propionibacterium acnes,* can be faced with imbalances or dysbiosis due to various factors.

When dysbiosis occurs in the skin, probiotics can act as modulators, restoring balance at the level of the skin microbiota. Over the last decade, the use of new technologies has facilitated the taxonomic analysis of the skin microbiota, whose bacterial population can amount to about 1010 species of microorganisms, belonging to more than 25 *phila,* among which the most represented are *Actinobacteria, Firmicutes,* and *Proteobacteria.* Various skin diseases have been linked to alterations of the skin microbiome, for example *P. acnes* is considered the pathogenic bacterium associated with skin acne. Therefore, maintaining a situation of homeostasis of the skin microbiota or restoring said homeostasis after a microbiota dysbiosis is fundamental for preventing or treating skin diseases, in particular skin infections or inflammations, even more particularly infections or inflammations due to pathogenic agents, such as bacteria or viruses as well as due to irradiation of the skin with UV rays or due to exposure of the skin to unfavourable conditions.

Classically, these problems are approached with the use of antibacterial agents, i.e. using disinfectants and topical antibiotics. On the one hand, though undoubtedly effective, besides eliminating beneficial bacteria, antibiotics pose a risk of sensitization and potential adverse events, particularly with the prolonged use of broad-spectrum antibiotics.

Furthermore, a conventional approach for the prevention of problems of damage or skin aging due to irradiation of the skin with UV rays is to use creams or skin products that reflect or absorb UV rays, commonly called sun filters. The active ingredients in so-called "chemical" solar filters (salicylates, cinnamates, *oxybenzone, octylcrylene* and others), thanks to their structure, are capable of absorbing UV light. On the other hand, titanium dioxide and zinc oxide are inert mineral substances with a strong covering power, which physically reflect the sunlight and which therefore are included among the so-called physical screens.

However, said solar filters only allow to prevent the interaction of UV rays with the skin, while they do not stimulate the maintenance of the homeostasis of the skin microbiota, nor restore said homeostasis in case of dysbiosis or infections or inflammations caused by said UV rays which may not be totally shielded by the solar filters.

Following an intense research and development activity, the Applicant addressed and solved the aforementioned technical problems by providing innovative compositions (in short, composition/s of the invention) comprising a mixture (in short, mixture of the invention) comprising or, alternatively, consisting of a live and viable or inactivated bacterial strain, belonging to the species *Lactobacillus* paracasei identified as *Lactobacillus paracasei* LPC-S01 DSM 26760 and hyaluronic acid or a salt thereof (in short, HA).

### SUMMARY OF THE INVENTION

A first aspect of the present invention relates to the use of a composition comprising probiotics and hyaluronic acid or a salt thereof to prevent, attenuate or treat natural skin aging or skin aging caused by exposure to external factors, such as for example UV rays.

A second aspect of the present invention relates to the use of a composition comprising probiotics and hyaluronic acid or a salt thereof to prevent, attenuate or treat at least one sign or symptom associated with or caused by a reduction in the immune defences of the skin.

A third aspect of the present invention relates to the use of a composition comprising probiotics and hyaluronic acid or a salt thereof to prevent, attenuate or treat at least one sign or symptom associated with or caused by an inflammation/infection of the skin.

The probiotics particularly useful for the purpose of the invention are bacteria belonging to the genus *Lactobacillus,* preferably to the species *Lactobacillus paracasei,* such as the strain L. paracasei LPC-S01 DSM 26760.

The Applicant actually found that a composition comprising probiotics, preferably bacteria belonging to the genus *Lactobacillus,* and hyaluronic acid is capable of enhancing the immune defences by favouring the release of antimicrobial peptides and chemokines of the skin, enhancing the normal process of differentiation and cell replacement of the epidermis, and reinforcing the structure of the dermis.

Furthermore, the Applicant found that the use of a composition comprising hyaluronic acid and probiotics, preferably bacteria belonging to the genus *Lactobacillus,* is capable of performing an anti-inflammatory action and preventing the activation of inflammation induced or caused by UV rays.

In particular, the compositions and mixtures of the present invention are capable of:
- inducing mechanisms for the defence and/or healing of the skin, in particular immunomodulatory mechanisms;
- preventing and/or treating inflammatory or infectious skin conditions, in particular those induced by UV radiation, both UV-A and UV-B;
- combating the proliferation of bacteria pathogenic to the skin;
- maintaining or restoring a homeostasis condition of the skin and/or a balance of the skin microbiota;
- favouring, facilitating, promoting and/or accelerating wound healing processes and/or reepithelization and/or cicatrization processes;
- combatting and/or slowing the skin aging.

Furthermore, the compositions of the invention are capable of inducing and/or promoting said positive effects both in the short and in the long term.

In addition, the mixtures and compositions of the invention do not have significant adverse effects and they can be administered to all subjects, particularly to paediatric subjects and pregnant or breastfeeding women.

Lastly, the mixtures and/or compositions of the invention are easy to prepare and cost-effective.

These and other objects, which will be clear from the detailed description that follows, are attained by the mixtures and compositions (containing said mixtures) of the present invention thanks to the technical characteristics claimed in the attached claims.

### BRIEF DESCRIPTION OF THE FIGURES

The present invention will be described in detail and exemplified below with reference to the attached figures, wherein:
Figure 1 shows a 20x and 40x magnification image of sections of histological tissues treated respectively with a saline solution (A) and with a composition comprising the strain LPC-S01 (B), marked with a Masson's trichrome stain;
Figure 2 shows a 20x and 40x magnification image of sections of histological tissues marked with a Masson's trichrome stain, treated respectively with a composition comprising hyaluronic acid (A) and with a composition comprising the strain LPC-S01 + hyaluronic acid (B);
Figure 3 shows the quantification of nuclear translocations of NFκB in tissues exposed to UV radiation and treated with a composition comprising the strain LPC-S01 (P1), with a composition comprising the strain LPC-S01 + hyaluronic acid (P2) and with a composition comprising hyaluronic acid (P3);
Figure 4 shows a 20x magnification image of sections of histological tissues treated respectively with a saline solution (A), with a composition comprising the strain LPC-S01 (B), with a composition comprising hyaluronic acid (C) and with a composition comprising the strain LPC-S01 and hyaluronic acid (D), 4 hours after UV insult, marked with a haematoxylin-eosin staining;
Figure 5 shows a 20x magnification image of sections of histological tissues treated respectively with a saline solution (A), with a composition comprising the strain LPC-S01 (B), with a composition comprising hyaluronic acid (C) and with a composition comprising the strain LPC-S01 and hyaluronic acid (D), after 24 hours of UV insult, marked with a haematoxylin-eosin staining.
Figure 6a, 6b, 6c, 6d: images acquired under the microscope of the histomorphological analysis by means of H&E staining in homeostasis model at 24 hours (6a: NC at 24 hours, 6b: P3-i at 24 hours) and at 48 hours (6c: NC at 48 hours, 6d: P3-i at 48 hours).
Figure 7: gene expression results (qRT-PCR) at 24 hours (RQ calculated with NC at 24 hours=1; RQ<0.5 de-regulation, RQ>2 upregulation) in homeostasis model for the P1-i, P2-i, P3-i products.
Figure 8: pre-treatment protocol with respect to the UV irradiation on "scratched" tissue with compositions of the invention comprising the inactivated strain.
Figure 9: post-treatment protocol with respect to the UV irradiation on "scratched" tissue with compositions of the invention comprising the inactivated strain.
Figures 10 and 11 show the reduction in viability of the pathogen C. *acnes* DMS1897 expressed in Log10 CFUs on skin inserts *in vitro* under the experimental conditions: exclusion model and competition model, respectively.

### DEFINITIONS

In the context of the present invention, the term "skin" is used to indicate the first line of defence with respect to the external environment; in particular, the defence is exercised through the action of keratinocytes scattered in the outer skin layer (epidermis) where they can induce the secretion of cytokines and chemokines to convey the warning message to the deeper layers of the skin, generating the inflammatory response.

In the context of the present invention, the expression "skin aging" is used to indicate an irreversible evolutionary process; it is expressed in a set of physiological alterations which determine the loss of skin hydration, the appearance of micro-wrinkles, the loss of elasticity, the hyperkeratosis and the formation of hyperpigmented spots, called "senile freckles".

In the context of the present invention, the term "probiotic" is used to indicate according to the definition given by the FAO and WHO: *"Live microorganisms which when administered in adequate amounts confer a health benefit on the host'.* In other words, probiotics are microorganisms (bacterial strains) that prove themselves capable of, once taken in appropriate quantities, to exert beneficial functions for the organism.

In the context of the present invention, the expression "hyaluronic acid" is used to indicate a glycosaminoglycan consisting of repeated units of glucosamine and glucuronic acid, bonded together, alternatively, by glycosidic bonds β1→4 and β1→3, as well as by intramolecular hydrogen bonds, which stabilise the conformations thereof.

### DETAILED DESCRIPTION OF THE INVENTION

Forming an object of the present invention is a composition (in short, composition of the invention or composition) comprising (I) a mixture M comprising, or alternatively, consisting of a bacterial strain belonging to the species *Lactobacillus paracasei* identified and deposited as *Lactobacillus paracasei* LPC-S01 DSM 26760 (live and viable or inactivated or a derivative thereof) and hyaluronic acid or a salt thereof; and (II) at least one acceptable pharmaceutical or cosmetic or food grade additive and/or excipient.

Forming an object of the present invention are said compositions or mixtures M of the invention, comprising or, alternatively, consisting of a bacterial strain belonging to the species *Lactobacillus* paracasei identified and deposited as the strain *L. paracasei* LPC-S01 DMS 26760 and hyaluronic acid or a salt thereof (according to any of the described embodiments), for use as medicament.

A first aspect of the present invention relates to a composition (composition of the invention) comprising probiotics (L. *paracasei* LPC-S01 DSM 26760, viable or inactivated or a derivative thereof), preferably probiotic bacteria, and hyaluronic acid or a salt thereof for use in the treatment (therapeutic or cosmetic), in the prevention or attenuation of at least one sign or symptom associated with or caused by skin aging (intrinsic aging or extrinsic aging, as defined below).

Said sign or symptom of skin aging is linked to a series of modifications which generally lead to thinning and/or yielding of the skin structure.

Preferably said aging is an intrinsic, or chronological, aging that depends substantially on genetic (or intrinsic) factors. Intrinsic aging, in principle, usually begins after 25 years of age. Preferably said sign or symptom associated with or caused by intrinsic skin aging is selected from among: wrinkles, skin laxity, loss or reduction of skin integrity, lack of skin elasticity, lack of skin tone, thinned skin, desquamation of the skin and skin dehydration, formation of dark spots or skin hyperpigmentation, also called "age spots".

Thus, forming an object of the present invention is the cosmetic use of the compositions or mixtures M of the invention, comprising or, alternatively, consisting of a bacterial strain belonging to the species *Lactobacillus paracasei* identified and deposited as the strain *L. paracasei* LPC-S01 DMS 26760 and hyaluronic acid or a salt thereof and, optionally, a first substance and/or second substance (according to any of the described embodiments), for the maintenance of homeostasis of the skin, and/or as anti-aging agent of the skin, for example for the cosmetic treatment of wrinkles, loss of elasticity of the skin (solar elastosis), dry or dehydrated skin, rough skin, photoaging, redness of the skin, presence of dilated capillaries on cheeks, nose and/or ears, sunspots, abnormal or uneven pigmentation or hyperpigmentation of the skin, or to make the skin brighter and more natural.

Alternatively, said skin aging is extrinsic, i.e. caused by external environmental factors (extrinsic factors). Extrinsic aging is for example caused by the aggression of external agents and/or environmental factors such as UV radiation (responsible for photoaging), cigarette smoking, alcohol abuse, pollution, continuous contact with irritants, cold, wind and combinations thereof. Photoaging is of particular interest in that it is related to numerous diseases or skin damage that can also lead to serious diseases, such as skin tumours.

Preferably said sign or symptom associated with or caused by extrinsic skin aging is selected from among: erythema, sun pigmentation or sunspots, keratosis, preferably hyperkeratosis, skin redness, sunburns, burns, photoaging, solar elastosis, cortical cataract, pterygium, reactivation of cold sores, skin damage of any nature (ulcer, wound or bruise), preferably lip and/or conjunctive damage, cutaneous melanoma, squamous skin carcinoma, basal cell carcinoma (basalioma), squamous corneal or conjunctiva carcinoma.

Skin aging is related to an alteration in the structure of the skin, which inevitably leads to increased susceptibility to inflammatory diseases and/or infections.

In an embodiment, the composition or mixture M of the invention comprising probiotics (L. *paracasei* LPC-S01 DSM 26760, live and viable or inactivated or a derivative thereof) and hyaluronic acid or a salt thereof is used to treat (therapeutic treatment method), to prevent or to attenuate at least one sign or symptom associated with or caused by a reduction in the immune system of the skin or inflammatory diseases and/or skin infections. In other words, the composition is used to enhance the immune defences of the skin.

Said at least one sign or symptom associated with or caused by a reduction in the immune defences of the skin or by inflammatory conditions of the skin is selected from among: dermatitis, preferably associated with irritation or excoriation, acne, acute or chronic dermatosis (e.g. rosacea or couperosa), skin infection, skin inflammation, erythema, ulcer, psoriasis, atopic dermatitis, otitis, rhagades, fistula and haemorrhoids.

Skin affections or diseases may be associated with, or caused by, pathogens. In this case the pathogens can be bacteria, fungi, yeasts, viruses and combinations thereof.

In an embodiment, the composition or mixture M of the invention comprising probiotics (L. *paracasei* LPC-S01 DSM 26760, viable or inactivated or a derivative thereof) and hyaluronic acid or a salt thereof is used to treat (therapeutic treatment method), to prevent or to attenuate at least one sign or symptom associated with or caused by skin inflammation/infection, for example associated with or caused by a pathogen agent.

Preferably said pathogens are bacteria, preferably bacteria of the genus *Propionibacterium,* preferably species *acnes* (*Propionibacterium acnes or Cutibacterium acnes,* in short C. acnes); *Staphylococcus,* preferably species *epidermidis, aureus, warneri, pyogenes, mitis; Corynebacterium ssp; Pseudomonas,* preferably species *aeruginosa; Acinetobacter,* preferably species *johnsonii*; *Streptococcus,* preferably species *pyogenes; Micrococcus ssp., Brevibacterium* ssp.

The experimental data prepared by the Applicant indicate that the composition or mixture M of the invention comprising hyaluronic acid and probiotics (L. *paracasei* LPC-S01 DSM 26760, live and viable or inactivated or a derivative thereof) is capable of exerting an anti-inflammatory and/or immunomodulating effect at the level of the keratinocytes and therefore of the skin.

Without wishing to be bound by any theory, the uses of the composition are due to the anti-inflammatory capacity, the immunomodulation, the renewal of the epidermis cells, the differentiation of the epidermis and the enhancement of the skin structure favoured by probiotics and by the hyaluronic acid contained in the composition.

In particular, the Applicant has shown that when the skin is exposed to the composition comprising hyaluronic acid and probiotics it is capable of increasing the differentiation process of the epidermis, in particular the stratum corneum became thicker. In addition, it was observed that collagen fibres were denser and more compact.

Furthermore, the composition of the invention comprising probiotics (L. *paracasei* LPC-S01 DSM 26760, viable or inactivated or a derivative thereof) and hyaluronic acid or a salt thereof is capable of exerting an immunomodulating (or immunostimulant) effect of the immune system of the skin, as is evident from the evaluation of the expression of coding genes for chemokines and defensins. In particular, an increase in the expression of defensin β2 has been observed both by probiotics and by the composition of the invention comprising hyaluronic acid and probiotics.

In an embodiment of the present invention, probiotics (present in the composition of the invention together with hyaluronic acid or a salt thereof) are preferably selected from among: bacteria, fungi, yeasts and combinations thereof; preferably bacteria, more preferably the bacterial strain belonging to the species *Lactobacillus paracasei* and identified as *L. paracasei* LPC-S01 DSM 26760 (live, inactivated or a derivative thereof).

According to a preferred aspect of the invention, the bacteria belong to at least one genus selected from among: *Lactobacillus, Bifidobacterium, Bacillus, Propionibacterium, Streptococcus, Lactococcus, Aerococcus* and *Enterococcus.*

More preferably the bacteria belong to the genus *Lactobacillus.*

According to a preferred further aspect of the invention, the bacteria of the genus *Lactobacillus* belong to at least one of the species selected from among: *Lactobacillus paracasei, Lactobacillus acidophilus, Lactobacillus amylolyticus, Lactobacillus amylovorus, Lactobacillus alimentarius, Lactobacillus aviaries, Lactobacillus brevis, Lactobacillus buchneri, Lactobacillus casei, Lactobacillus cellobiosus, Lactobacillus coryniformis, Lactobacillus crispatus, Lactobacillus curvatus, Lactobacillus delbrueckii, Lactobacillus farciminis, Lactobacillus fermentum, Lactobacillus gallinarum, Lactobacillus gasseri, Lactobacillus helveticus, Lactobacillus hilgardii, Lactobacillus johnsonii, Lactobacillus kefiranofaciens, Lactobacillus kefiri, Lactobacillus mucosae, Lactobacillus panis, Lactobacillus collinoides, Lactobacillus paraplantarum, Lactobacillus pentosus, Lactobacillus plantarum, Lactobacillus pontis, Lactobacillus reuteri, Lactobacillus rhamnosus, Lactobacillus sakei, Lactobacillus salivarius, Lactobacillus sanfrancisensis* and combinations thereof.

More preferably, the lactobacilli are of the species *Lactobacillus paracasei,* preferably the strain *Lactobacillus paracasei* DG^{®} CNCM I-1572 and/or the strain *Lactobacillus paracasei* LPC-S01 DSM26760.

Both strains were isolated and deposited by SOFAR S.p.A.; in particular, the bacterial strain L. *casei* DG ^{®} (trademark registered by Sofar, Italy) was deposited by SOFAR S.p.A. on May 5, 1995 at the National Collection of Cultures of Microorganisms of the Pasteur Institute in Paris under the deposit number CNCM I-1572. Initially the strain had the name *Lactobacillus casei* DG sub. casei (reclassified as *Lactobacillus paracasei* DG^{®} CNCM I-1572).

The bacterial strain *Lactobacillus paracasei* LPC-S01 was deposited at Deutsche Sammlung von Mikroorganismen und Zellkulturen under the accession number DSM 26760 on 15/05/2017 by SOFAR S.p.A. (date of application for conversion of the deposit in accordance with the Budapest Treaty; date of original deposit 11/01/2013).

The *Lactobacillus paracasei* strains were reclassified as *Lacticaseibacillus paracasei.*

In a preferred embodiment of the invention, the composition comprises *Lactobacillus paracasei* LPC-S01 and hyaluronic acid or a salt thereof.

According to a preferred further aspect of the invention, the bacteria of the genus *Bifidobacterium* belong to at least one of the species selected from among: *B. animalis, B. bifidum, B. breve, B. infantis, B. longum,* B. *adolescentis,* B. *catenulatum, B. angulatum, B. asteroides, B. boum, B. choerinum, B. coryneforme, B. cuniculi,* B. *denticolens, B. dentium, B. gallicum, B. gallinarum, B. indicum, B. inopinatum,* B. *lactis, B. magnum, B. merycicum, B. minimum, B. pseudocatenulatum, B. pseudolongum, B. pullorum, B. ruminantium, B. saeculare, B. subfile, B. thermacidophilum B. thermophilum e B. tsurumiense; more preferably selected from among:*

*Bacillus clausii, Bacillus subtilis, Bacillus coagulans, Bacillus megaterium Bacillus halodurans, Bacillus thuringiensis, Bacillus insolifus* e *Bacillus marinus.*

According to a preferred further aspect of the invention, the bacteria of the genus *Propionibacterium* belong to at least one of the species selected from among: *P. shermanii, P. acnes, P. australiense, P. avidum, P. cyclohexanicum, P. freudenreichii, P. granulosum, P. jensenii, P. microaerophilum, P. propionicum* and *P. thoenii.*

According to a preferred further aspect of the invention, the bacteria of the genus *Streptococcus* belong to at least one of the species selected from among: *Streptococcus thermophilus, Streptococcus salivarius, Streptococcus agalactiae, Streptococcus anginosus, Streptococcus bovis, Streptococcus canis, Streptococcus constellatus, Streptococcus downei, Streptococcus dysgalactiae, Streptococcus equinus, Streptococcus fetus, Streptococcus infantarius, Streptococcus iniae, Streptococcus intermedius, Streptococcus milleri, Streptococcus mitis, Streptococcus mutans, Streptococcus oralis, Streptococcus orisratti, Streptococcus parasanguinis, Streptococcus peroris, Streptococcus pneumoniae, Streptococcus pseudopneumoniae, Streptococcus pyogenes, Streptococcus ratti, Streptococcus tigurinus, Streptococcus sanguinis, Streptococcus sobrinus, Streptococcus suis, Streptococcus uberis, Streptococcus vestibularis, Streptococcus viridans* and *Streptococcus zooepidemicus.*

According to a preferred further aspect of the invention, the bacteria of the genus *Lactococcus* belong to at least one of the species selected from among: *L. chungangensis, L. formosensis, L. fujiensis, L. garvieae, L. lactis, L. piscium, L. plantarum, L. raffinolactis* and *L. taiwanensis.*

According to a preferred further aspect of the invention, the bacteria of the genus *Aerococcus* belong to at least one of the species selected from among: *A. urinae, A. sanguinicola, A. christensenii, A. suis, A. urinaeequi* and *A. urinaehominis.*

According to a preferred further aspect of the invention, the bacteria of the genus *Enterococcus* belong to at least one of the species selected from among: *Enterococcus avium, Enterococcus durans, Enterococcus faecalis, Enterococcus faecium, Enterococcus gallinarum, Enterococcus haemoperoxidus, Enterococcus hirae, Enterococcus malodoratus, Enterococcus moraviensis, Enterococcus mundtii, Enterococcus pseudoavium, Enterococcus raffinosus* and *Enterococcus solitarius.*

According to a further preferred aspect of the invention, the yeasts belong to the genus *Saccharomyces,* more preferably of the species *Saccharomyces cerevisiae* and/or *Saccharomyces boulardii.*

In the mixture or composition of the present invention, the probiotics, preferably the bacterial strain *L. casei* DG^{®} CNCM I-1572 and/or the bacterial strain *Lactobacillus paracasei* LPC-S01 DSM 26760, are used (together with hyaluronic acid or a salt thereof) live, that is, they are used as probiotics.

Alternatively, said probiotics, preferably the bacterial strain *L. casei* DG^{®} CNCM I-1572, and/or the bacterial strain *Lactobacillus paracasei* LPC-S01 DSM 26760 are dead and/or inactivated and/or tyndallized.

For example, the viable bacterial strains (probiotics) of the present invention (*e.g. L. paracasei* LPC-S01 DSM 26760) can be inactivated by heating or tyndallization or gamma irradiation or sonication. Said inactivation by heating or tyndallization can be carried out at a temperature comprised in the range of from 50°C to 120°C, preferably from 65°C to 105°C, more preferably from 75°C to 95°C, for example about 85°C, for a time comprised in the range from 30 minutes to 120 minutes, preferably from 45 minutes to 85 minutes, for example about 60 minutes; or, alternatively by means of tyndallization. The heating or tyndallization or gamma irradiation or sonication process is carried out according to the techniques, procedures and apparatuses known to the man skilled in the art.

The bacteria subjected to said inactivation process by heating or tyndallization are dead (control by means of plate count and/or cytofluorimetry) while the cell wall remains intact, preferably at a % of bacteria comprised in the range from 70% to 99.5%, preferably from 80% to 95%, with respect to the total number of bacteria subjected to said heating or tyndallization inactivation techniques.

In a further embodiment, the probiotics, preferably the bacterial strain *L. casei* DG^{®} CNCM I-1572 and/or the bacterial strain *Lactobacillus* paracasei LPC-S01 DSM 26760, are used (together with hyaluronic acid or a salt thereof) in the form of lysate and/or extract, that is, they are used as paraprobiotic.

Alternatively, said probiotics, preferably the bacterial strain L. casei DG^{®} CNCM I-1572 and/or the bacterial strain *Lactobacillus paracasei* LPC-S01 DSM 26760, are used (together with hyaluronic acid or a salt thereof) in the form of bacterial products selected from among: supernatant, metabolites, metabolic bioproducts, postbiotics, cell wall and components thereof, exopolysaccharide, ribosomes and glycoproteins, glucans and other polysaccharides, lipopolysaccharides and any component of the supernatant.

In short, in the context of the present invention, the expression "derivative/s" of a bacterial strain or of a viable bacterial strain or probiotics (*e.g. L. paracasei* LPC-S01 DSM 26760) the aforementioned paraprobiotics (lysates or extracts) or any derivative and/or component of the bacterial strain (supernatant, metabolites, metabolic bioproducts, postbiotics, cell wall and components thereof, exopolysaccharide, ribosomes and glycoproteins, glucans and other polysaccharides, lipopolysaccharides or any component of the supernatant) which, if administered (through the oral or topical route) in appropriate quantities, confer a benefit to the human or animal consumer.

In the context of the present invention, the term "probiotics", unless otherwise specified, indicates and comprises bacterial strains (*e.g. L. paracasei* LPC-S01 DSM 26760), live and inactivated, and derivatives of said bacterial strains, as defined above.

In general, said probiotics are single microorganisms or combinations of microorganisms, or consortia, of any microbial species listed in EFSA's QPS list.

Preferably, the composition as described above comprises a combination of the strains reported above with other microorganisms as described above, preferably selected from among: bacteria, fungi, yeasts and combinations thereof.

Probiotics, preferably the bacterial strain *L. casei* DG^{®} CNCM I-1572 and/or the bacterial strain *Lactobacillus paracasei* LPC-S01 DSM 26760, are present in the composition (together with hyaluronic acid or a salt thereof) at a minimum amount sufficient to allow temporary colonisation of the skin, bowel and/or other regions of the organism. Preferably said amount varies at a concentration comprised in the range from 1×10⁶ CFU to 1×10¹² CFU, preferably between 10⁸ and 10¹² units of microorganisms, more preferably 10⁹ and 10¹¹ units of microorganisms, for example about or above 1×10⁹ CFU *(Colony Forming Unit),* with respect to the daily intake (or single dose) of composition of the invention.

The probiotics of the present invention, preferably the bacterial strain *L. casei* DG^{®} CNCM I-1572 and/or the bacterial strain *Lactobacillus paracasei* LPC-S01 DSM 26760, are preferably administered at an amount variable between 10⁸ and 10¹² units of microorganisms, more preferably between 10⁹ and 10¹¹ units of microorganisms, for each intake.

According to a preferred aspect, the intake of probiotics, preferably bacteria, is carried out at least 1-2 times a day.

As described above, the composition or mixture M of the invention comprises hyaluronic acid or a salt thereof and combinations thereof. Said hyaluronic acid or a salt thereof (in short, HA), comprised in the composition of the invention together with the strain *L. paracasei* LPC-S01 DMS 26760 and, optionally, at least one further first or second substance (according to any of the described embodiments), preferably has an average molecular weight comprised in the range from about 10 kDa (kiloDalton=1,000.00 Dalton or 10³ Dalton) to 3,000 kDa, preferably from about 800 kDa to 2,500 kDa, more preferably from about 1,000 kDa to 2,000 kDa, for example about 1,300 kDa, 1,400 kDa, 1,500 kDa, 1,600 kDa, 1,700 kDa, 1,800 kDa. In an embodiment, the hyaluronic acid salt (for example, alkaline or alkaline earth metal hyaluronate) is selected from among: sodium hyaluronate, potassium hyaluronate, ammonium hyaluronate, calcium hyaluronate, magnesium hyaluronate, zinc hyaluronate, cobalt hyaluronate and combinations thereof.

Furthermore, in the context of the present invention, the term "hyaluronic acid or a salt thereof" or "HA" or simply "hyaluronic acid" is used to indicate hyaluronic acid as such, and hydrolysed hyaluronic acid (for example obtained by fermentation), as well as a hyaluronic acid salt (hyaluronate) as described above.

When said hyaluronic acid of the invention is a biotechnological hydrolysed hyaluronic acid obtained by fermentation, it can have an average molecular weight of about 10 kDa. When said hyaluronic acid of the invention is a sodium hyaluronate (for example CAS N° 9067-32-7, Mw - *Molecular weight* 1,000-1,400 kDa or Mw 1,000-1,700 kDa) or potassium hyaluronate, it may have an average molecular weight comprised in the range from about 1,000 kDa to 2,000 kDa.

Besides *L. paracasei* LPC-01 DSM 26760 and HA, the composition of the invention advantageously further comprises pharmaceutically accepted excipients and/or further substances (for example, a first substance or second substance as described below) and/or carriers.

Besides *L. paracasei* LPC-01 DSM 26760 and HA and, optionally, a second substance (defined below), the composition of the present invention preferably further comprises a first substance selected from among: plasma, PRP, cicatrizing substances, re-epithelizing substances, humectants, hydrating agents, emollient agents, adsorbing agents, analgesics, phlebotonics, anti-inflammatory agents, muscle relaxants, antibiotics, antibacterial agents, antimycotics, antivirals, pesticides, peptide and/or protein substances and/or proteins, such as collagen, substances belonging to connective tissue such as glycosaminoglycans, preferably chondroitin sulphate, and/or combinations thereof.

In an embodiment of the present invention, the composition is formulated (for example in solid, semi-solid or liquid form) for topical or cutaneous topical applications, preferably in the form of cream, gel, oil, emulsions (or foam), solutions, dispersions (solid-liquid or liquid-liquid), suspensions, biphasic mixtures, sprays (spray liquids), gauzes, plasters, bandages, lotions, mousse, masks (masks applicable to the skin), ointments or pastes.

In an embodiment of the present invention, the composition is formulated for oral administration, preferably as a tablet, capsule, bar, granular powder, operculum, buccal soluble granule, sachet or pill, suspensions (for example drinkable phials) or solutions (monophasic or biphasic). Alternatively, the composition is prepared extemporaneously by mixing the composition with water. Alternatively, the composition is prepared extemporaneously by mixing together the two components, probiotics (*L. paracasei* LPC-01 DSM 26760) and hyaluronic acid or a salt thereof, for example for the preparation of masks to be applied to the skin or suspensions for oral use (drinkable phials). In particular, the device for the extemporaneous preparation of the composition of the invention may consist of a phial comprising an aqueous solution of hyaluronic acid or a salt thereof and a portion at one end of the bottle (*e.g.* to seal the phial, such as a cap) comprising the bacterial strain (*L. paracasei* LPC-01 DSM 26760, viable or inactivated or derivative thereof) therein in solid form of powder, granules or tablet; the extemporaneous preparation of the composition of the invention occurs by releasing (*e.g.* by pressure) the bacterial strain in solid form from the portion at the end of the phial to the solution of hyaluronic acid or salt thereof comprised in the phial.

According to a preferred embodiment, besides *L. paracasei* LPC-01 DSM 26760 and HA and, optionally, said first substance, the composition of the invention comprises other substances (second substance) selected from among: amino acids, supplements, vitamins, trace elements such as zinc and selenium, macro and micronutrients, enzymes and/or prebiotic substances such as fructooligosaccharides (FOS), galacto-oligosaccharides (GOS), xylo-oligosaccharides (XOS), inulin, guar gum or combinations thereof.

The composition of the invention, comprising *L. paracasei* LPC-01 DSM 26760, HA and, optionally, a first or second substance, further comprises said (II) at least one pharmaceutical or food or cosmetic grade additive and/or excipient, that is a substance devoid of therapeutic activity suitable for pharmaceutical or food use. In the context of the present invention, the additives and/or excipients acceptable for pharmaceutical or food or cosmetic use comprise all the auxiliary substances known to the man skilled in the art for the preparation of compositions in solid, semi-solid or liquid form, such as, for example, diluents, solvents (including water, glycerine, ethyl alcohol), solubilizers, acidifiers, thickeners, sweeteners, flavour enhancers, colourants, lubricants, surfactants, preservatives, pH stabilizing buffers and mixtures thereof.

The compositions of the invention, comprising the strain *L. paracasei* LPC-S01 DMS 26760 and hyaluronic acid or a salt thereof and, optionally, a first and/or second substance, can be pharmaceutical compositions (or *Live Biotherapeutic Products),* medical device compositions, dietary supplements, foods, *novel foods,* probiotic products, compositions for a food for special medical purpose (FSMP), or cosmetic compositions.

Embodiments (FRn) of the present invention are outlined below:
FR1. Composition comprising probiotics and hyaluronic acid or a salt thereof for use in the treatment, in the prevention or in the attenuation of at least one sign or symptom associated with/caused by skin aging or in the treatment, in the prevention or in attenuation of at least one sign or symptom associated with/caused by a reduction in the immune system of the skin.
FR2. The composition for use according to FR1, where skin aging is selected from among intrinsic skin aging and extrinsic skin aging.
FR3. The composition according to FR2, wherein said at least one sign or symptom associated with/caused by intrinsic skin aging is selected from among: wrinkles, skin laxity, loss or reduction of skin integrity, lack of skin elasticity, lack of skin tone, thinned skin, desquamation of the skin and skin dehydration.
FR4. The composition for use according to FR2, wherein the at least one sign or symptom associated with/caused by extrinsic skin aging is selected from among: erythema, pigmentation, keratosis, preferably hyperkeratosis, skin redness, burns, cortical cataract, pterygium, reactivation of cold sores, skin damage of any nature, preferably damage to the lips and/or conjunctive, cutaneous melanoma, squamous carcinoma of the skin, basal cell carcinoma (basalioma), squamous carcinoma of the cornea or conjunctiva.
FR5. The composition for use according to FR1, wherein the at least one sign or symptom associated with/caused by a reduction in the immune system of the skin is selected from among: dermatitis, preferably associated with irritation or excoriation, acne, infection, skin inflammation, erythema, ulcer, psoriasis, atopic dermatitis, otitis, rhagades, fistula and haemorrhoids.
FR6. The composition for use according to any one of FR1-5, wherein said probiotics are preferably selected from among: bacteria, fungi, yeasts and combinations thereof, preferably they are bacteria belonging to at least one genus selected from among: *Lactobacillus, Bifidobacterium, Bacillus, Propionibacterium, Streptococcus, Lactococcus, Aerococcus* and *Enterococcus.*
FR7. The composition for use according to FR6, wherein the bacteria of the genus *Lactobacillus* belong to at least one of the species selected from among: *Lactobacillus paracasei, Lactobacillus acidophilus, Lactobacillus amylolyticus, Lactobacillus amylovorus, Lactobacillus alimentarius, Lactobacillus aviaries, Lactobacillus brevis, Lactobacillus buchneri, Lactobacillus casei, Lactobacillus cellobiosus, Lactobacillus coryniformis, Lactobacillus crispatus, Lactobacillus curvatus, Lactobacillus delbrueckii, Lactobacillus farciminis, Lactobacillus fermentum, Lactobacillus gallinarum, Lactobacillus gasseri, Lactobacillus helveticus, Lactobacillus hilgardii, Lactobacillus johnsonii, Lactobacillus kefiranofaciens, Lactobacillus kefiri, Lactobacillus mucosae, Lactobacillus panis, Lactobacillus collinoides, Lactobacillus paraplantarum, Lactobacillus pentosus, Lactobacillus plantarum, Lactobacillus pontis, Lactobacillus reuteri, Lactobacillus rhamnosus, Lactobacillus sakei, Lactobacillus salivarius, Lactobacillus sanfrancisensis* and combinations thereof.
FR8. The composition for use according to FR6 or FR7, wherein the bacteria are of the species *Lactobacillus paracasei,* preferably the strain *Lactobacillus paracasei* DG^{®} CNCM I-1572 and/or the strain *Lactobacillus paracasei* LPC-S01 DSM 26760.
FR9. The composition for use according to any one of the preceding FRs, wherein the hyaluronic acid salt is selected from among: sodium hyaluronate, potassium hyaluronate, ammonium hyaluronate, calcium hyaluronate, magnesium hyaluronate, zinc hyaluronate, cobalt hyaluronate and combinations thereof.
FR10. The composition for use according to any one of the preceding FRs, wherein said probiotics are live and/or dead and/or inactivated and/or tyndallized, and/or in the form of lysate and/or extract, and/or in the form of bacterial products selected from among: supernatant, metabolites, metabolic bioproducts, postbiotics, cell wall and its components, exopolysaccharide, ribosomes and glycoproteins, glucans and other polysaccharides, lipopolysaccharides and any component of the supernatant.
FR11. The composition for use according to any one of the preceding FRs, wherein said probiotics are present at an amount variable between 10⁸ and 10¹² units of microorganism, preferably 10⁹ and 10¹¹ units of microorganism.
FR12. The composition for use according to any of the preceding FRs in the form of cream, gel, oil, emulsions, sprays, gauzes, plasters, bandages, lotions, mousse, masks, ointments, pastes or liquid formulations for extemporaneous preparation.

The term "treatment" or "therapeutic treatment" or "treatment method" in the context of the present invention is used to indicate an intervention on a subject in need, comprising the administration of a therapeutically effective amount of the composition or mixture M of the invention, for the purpose of elimination, the reduction/decrease or prevention of a pathology or disease and the symptoms or disorders thereof.

The term "therapeutically effective amount" refers to the amount of active compound and/or bacterial strain that elicits the biological or medicinal response in a tissue, system, mammal, or human being that is sought and defined by an individual, researcher, veterinarian, physician, or other clinician or health worker.

In the context of the present invention, the expression "subjects" is used to indicate human subjects or animal subjects (e.g. pets, such as dogs or cats or other mammals). Preferably, the compositions of the invention are for use in methods of medical treatment or for cosmetic use on human subjects.

In the context of the present invention, the term "medical device" is used in the meaning according to the Legislative Decree n° 46 dated 24 February 1997, or in accordance with the new Medical device regulation (EU) 2017/745 (MDR).

In the context of the present invention, the term"novel *food*" is used in the meaning according to Regulation EC 258 dated 1997.

Unless otherwise specified, the expression composition or mixture or other comprising a component at an amount "comprised in a range from x to y" is used to indicate that said component may be present in the composition or mixture or extract or other at all amounts present in said range, even if not specified, extremes of the range comprised.

### EXAMPLE

### Example-A

Cream according to the invention comprising the strain *L. paracasei* LPC-S01 DMS 26760 and hyaluronic acid or a salt thereof:
to prepare said cream, the strain LPC-S01 DSM 26760 (viable or inactivated) - freeze-dried in the form of a powder or powder capsule (about 8×10⁹ CFU) - was dissolved in 13 ml of a hyaluronic acid-based cream (in short, HA cream).

Example of composition of the HA cream (% weight/weight):
- water (solvent) q.s. at 100%;
- functional substances: octocrylene (UV-B filter) 2-8% (4.5%), encapsulated BMDBM stabilized with octocrylene (UV-B filter) 0.1-5% (2%), shea organic butter (butyrospermum parkii = shea) (CAS N° 194043-92-0) 0.1-4% (1%), collagen complex and panthenol 0.1-4% (1%), butyl methoxydibenzoylmethane (UV-A filter) 0.1-4% (1%), vitamin E 0.01-1% (0.3%), gardenia jasminoide stems 0.01-1% (0.1%), low molecular weight hyaluronic acid (e.g. CAS N° 9004-61-9) 0.005-1% (0.05%), medium/high hyaluronic acid (e.g. CAS N° 9067-32-7) 0.005-1% (0.02%);
- excipients and additives (2-10% w/w, according to technical requirements known to the man skilled in the art): antioxidants (e.g. tocopheryl acetate), preservatives (e.g. phenoxyethanol, potassium sorbate), surfactants-emulsifiers (e.g. cetearyl glucoside, cetyl alcohol), emulsion stabilizer (e.g. carbomer), solvents (e.g. 1,2-hexanediol), abrasives (e.g. silica), binder (e.g. PVP), humectant (e.g. glycerine), and/or skin conditioning agents (e.g. dimethicone, xanthan gum, tropolone).

### Example-B

Mask according to the invention comprising the strain *L. paracasei* LPC-S01 DMS 26760 and hyaluronic acid or a salt thereof:
to prepare said mask, the strain LPC-S01 DSM 26760 (viable or inactivated, preferably viable) - freeze-dried in the form of a powder or powder capsule (about 8×10⁹ CFU) - was dissolved in an aqueous solution of hyaluronic acid or a salt thereof, for example, a solution having the components reported in Table A, in which low molecular weight hyaluronic acid (e.g. CAS N° 9004-61-9) 0.01-2% (0.1%) and medium/high molecular weight hyaluronic acid (e.g. CAS N° 9067-32-7) 0.005-1% (0.05%) are present at % by weight/weight (on the total weight of said aqueous solution of hyaluronic acid) .

**Table A**

| Ingredient | CAS N° |
|---|---|
| water | 7732-18-5 |
| propanediol | 504-63-2 |
| sodium hyaluronate | 9067-32-7 |
| phenoxyethanol | 122-99-6 |
| 1,2-hexanediol | 6920-22-5 |
| caprylyl glycol | 1117-86-8 |
| hydrolysed hyaluronic acid | 9004-61-9 |
| sodium hydroxide | 1310-73-2 |
| additives/excipients | - |

### - Strain L. paracasei LPC-S01 DMS 26760 inactivated by heating:

### EXPERIMENTAL PART - (1)

### Episkin T-Skin^{™} MODEL (3D skin model)

Episkin T-Skin^{™} (in short, T-Skin or "Full thickness Skin" or 3D Skin) is a 3D skin model reconstructed *in vitro,* including the dermis and epidermis (full thickness skin model).

Episkin T-Skin^{™} is an in vitro reconstructed skin consisting of a skin equivalent with human fibroblasts superimposed on a well differentiated stratified epidermis derived from normal human keratinocytes cultured on an inert polycarbonate filter.

In vitro reconstructed human skin models are closer - in terms of morphology (multi-layered epithelium), biochemical and physiological properties - to in vivo human tissues and they are now the most promising alternative to animals, to ex *vivo* explants and to submerged cell monolayers for the evaluation of efficacy and safety of topical application of products (Gordon et al. 2015, Zuang V. 2016).

The biological relevance and predictivity of these models derive from the presence of a tissue organized with different layers of living cells that allows to evaluate products topically applied at realistic clinical doses and exposure conditions. The treatment of human skin with locally applied products, such as cosmetics, leads to a genomic response that has a dynamic pathway and it represents the first cellular signal, at the transcription level, responsible for a cascade of events. 3D human tissues are relevant test systems for studying the mechanism of action and evaluating the efficacy of a product taking in to account both the direct genomic response and the results of cell and crosstalk communication through soluble mediators and the expression of specific biomarkers.

### I. HOMEOSTATIC MODEL (non-inflammatory) on Episkin T-Skin^{™} model

In the present in vitro study of homeostatic model on the Episkin T-Skin^{™} model, the efficacy in terms of benefits for homeostasis of the skin of compositions according to the invention comprising hyaluronic acid (HA) and a viable or inactivated bacterial strain LPC-S01 DSM 26760 was evaluated to explore its potential application and efficacy for skin care.

The purpose of the study is to study the skin tolerance profile of compositions according to the invention after exposure to high concentrations and to evaluate their efficacy at:
- enhancing of skin self-defence by means of induction of antimicrobial peptides,
- innate immune-response stimulation of keratinocytes, epidermal renewal and differentiation,
- inducing a positive renewal of the epidermal and dermal compartments, acting as anti-aging agent.

### 1.1. VIABLE bacterial strain - HOMEOSTATIC MODEL

### 1.1.1. Products under analysis and Control.

- Negative Control (NC): 0.9% NaCl saline solution.
- P1: viable strain LPC-S01 DSM 26760 resuspended in saline solution;
- P2: hyaluronic acid-based aqueous solution (as in Example-B (mask) reported above);
- P3: viable strain LPC-S01 DSM 26760 resuspended in hyaluronic acid-based aqueous solution;

To prepare P3, the content of a powder capsule (about 8×10⁹ CFU) of the freeze-dried viable strain LPC-S01 DSM 26760 was dissolved in 13 ml of a hyaluronic acid-based aqueous solution.

To prepare P1 (strain only), the content of a powder capsule (about 8×10⁹ CFU) of a freeze-dried viable strain LPC-S01 DSM 26760 was resuspended in 13 ml of saline solution.

### 1.1.2. Methodology Evaluation of the increase in self-defence and cell renewal capacity

The increase in the self-defence and cell renewal capacity of the probiotic strain LPC-S01 *(Lactobacillus paracasei* LPC-S01 DSM 26760, viable; P1), of the probiotic strain LPC-S01 (viable) + hyaluronic acid (P3) and hyaluronic acid (P2) was evaluated on an *in vitro* reconstructed full 3D skin model that reproduces the compartments of the dermis and epidermis and therefore allows to study the modifications of the extracellular matrix of the dermis and the differentiation of viable skin layers ("Full thickness skin" model).

The study focused on the evaluation of the effect of the strain LPC-S01 DSM 26760, hyaluronic acid and a composition comprising the strain LPC-S01 + hyaluronic acid on the activation of an immune response, cell differentiation of the epidermis and cell renewal of the dermis. The strain LPC-S01 DSM 26760, hyaluronic acid and the composition comprising the strain LPC-S01 probiotic + hyaluronic acid were applied (30 µl) directly to the surface of the 3D skin model, incubated for 8 hours and then rinsed using saline solution to eliminate the excess product. Tissues were cultured for a further 16 hours prior to analysis, to mimic a realistic exposure of a face mask in 24 hours.

A saline solution was used as the negative control (NC).

The following parameters were analysed with respect to the negative control (according to methods known to the man skilled in the art):
- Histological analysis by means of Masson's Trichrome staining;
- Gene expression (qRT-PCR) of the main biomarkers of skin defence (HBD2 and CCL27), innate immune response (TLR2), epidermal differentiation (KRT14, LOR and IVL), epidermal renewal (HAS-2, CD44, Collagen III, IV and XIII, KGF and EGF);
- Cell damage by quantifying the release of adenylate kinase (in short, AK) (Toxilight assay).

The experiments were carried out in biological triplicate.

### I.1.2.1. Histomorphologica analysis by means of Masson's trichrome staining

At the end of the treatment the tissues were washed with saline solution and fixed in 10% formalin. The tissue sections were marked using the Masson Trichrome kit (Abcam 150686) according to the manufacturer's instructions. For each sample, 3 microscopic acquisitions were performed on 3 different parts of the section. Histological samples were analysed under an optical microscope (20x and 40x magnification) and morphological modifications of the tissue were evaluated.

### 1.1.2.2. Real time PCR analysis

At the end of the treatment, tissues were collected in lysis buffer for RNA extraction and cDNA reverse transcription. RNA integrity was evaluated by loading the RNA extracted in 1% agarose gel: 18S and 28S ribosomal bands were detected. GAPDH was used as an endogenous control gene to normalise input amounts. The analysis of the data obtained was carried out using methods known to the man skilled in the art.

### 1.1.3. Results of the activation of in self-defence and cell renewal capacity

### I.1.3.1. Results of histomorphological analysis by means of Masson's Trichrome staining.

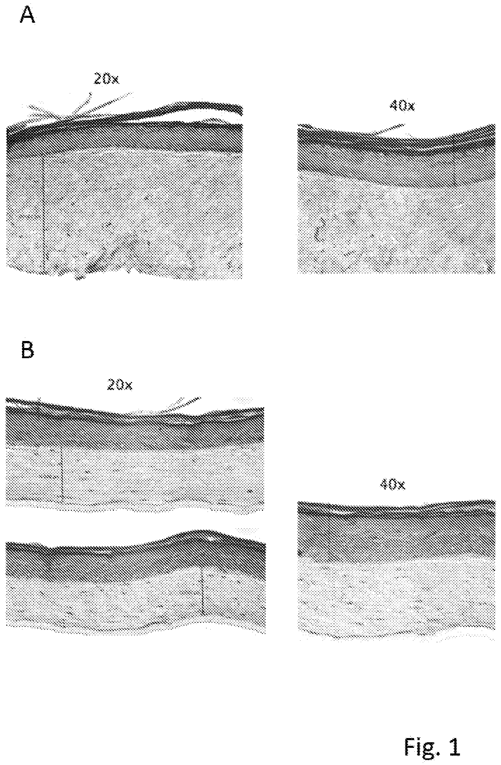

After incubation with the saline solution, the negative control (Figure 1A) showed no morphology and/or structural changes in both the epidermis and in the dermis. The distribution of collagen fibres was oriented and comprised fibroblasts.

Treatment with the strain LPC-S01 DSM 26760 (viable) did not induce significant alterations in the structure of the epidermis and dermis. The collagen fibres and their distribution showed a reduced thickness and a reduced density of the fibres with respect to the negative control (Figure 1B).

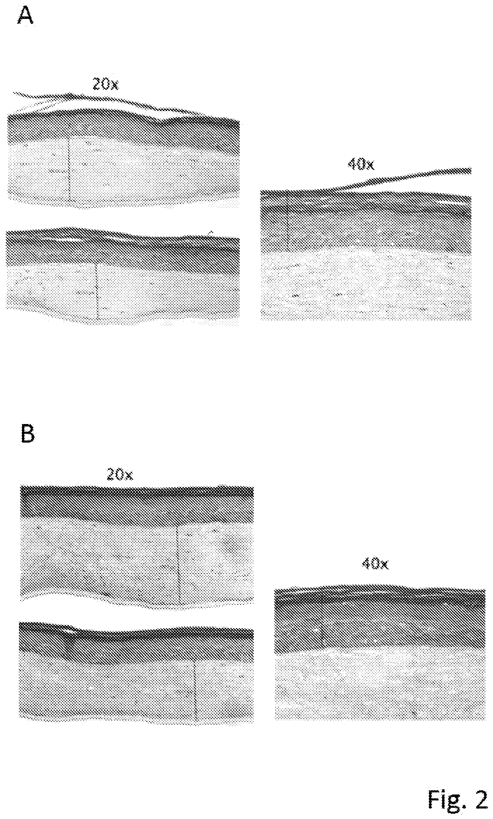

Treatment with hyaluronic acid did not induce significant alterations in the structure of the epidermis and dermis. Collagen fibres showed a reduced density compared to the negative control (Figure 2A).

The treatment with the probiotic strain LPC-S01 DSM 26760 (viable) + hyaluronic acid was capable of modifying the morphology of the epidermis, in particular of the stratum corneum which was thinner, increasing the differentiation process (Figure 2B).

### I.1.3.2. Real time PCR results (qRT-PCR).

Table 1 shows the relative quantification (RQ), with respect to the negative control, of the values obtained by means of Real time PCR, of the tissues treated with LPC-S01 DSM 26760 (viable), hyaluronic acid and LPC-S01 DSM 26760 (viable)+ hyaluronic acid.

**Table 1**

| **Compartment** | **Function** | **Biomarker** | **LPC-S01** | **Hyaluronic acid** | **Hyaluronic acid + LPC-S01** |
|---|---|---|---|---|---|
| Epidermis | Skin defense | Human defensin β2 | 5.80 | 0.59 | 2.18 |
| | | CCL27 | 1.12 | 0.87 | 0.96 |
| | Innate immune response | TLR2 | 1.94 | 0.93 | 1.38 |
| | Epidermis differentiation | Cytokeratin 14 | 1.11 | 0.96 | 1.23 |
| | | Loricrin | 0.59 | 1.35 | 1.68 |
| | | Involucrin | 1.50 | 0.98 | 1.30 |
| | Epidermis structure | Collagen XIII | 0.91 | 1.06 | 0.92 |
| | | KGF | 0.61 | 1.08 | 0.37 |
| | | EGF | 0.95 | 1.09 | 1.61 |
| Dermis | Cell renewal, *anti-aging,* cellular matrix | HAS-2 | 0.53 | 1.08 | 0.53 |
| | | CD44 | 1.09 | 1.00 | 1.10 |
| | | Collagen III | 0.45 | 0.75 | 0.33 |
| | | Collagen IV | 1.00 | 1.01 | 0.12 |

Treatment with probiotic LPC-S01 DSM 26760 induces an increase in human defensin β2 (HBD2) and TLR2. These data are consistent with the immunomodulatory properties of the probiotic, and they indicate its ability to trigger skin defences and enhance the innate immune response of the skin.

Treatment with hyaluronic acid cannot induce a modulation of the expression of the genes subject of evaluation.

Treatment with hyaluronic acid + LPC-S01 DSM 26760 causes an increase in HBD2 expression compared to treatment with hyaluronic acid. A decrease in Collagen III and HAS-2 values was observed (as already observed in probiotic treatment alone), while a reduction in KGF was also observed.

Generally speaking, the results obtained indicate that when applied alone the probiotic LPC-S01 DSM 26760 exerts a positive effect on the skin by reinforcing its innate immunity (based on TLR2 and HBD-2).

Hyaluronic acid applied alone does not exert a positive effect on the skin; on the contrary, the LPC-S01 introduced in the same formulation with hyaluronic acid is capable of maintaining its main activity in enhancing the innate defence of the skin.

### I.1.3.4 Result of the release of adenylate kinase (Toxilight assay).

Levels of adenylate kinase released by tissues treated with the products subject of study indicate good biocompatibility of the products after 8 and 16 hours of incubation.

### I.1.4. Conclusions

The results obtained show that the treatment with hyaluronic acid + LPC-S01 DSM 26760 (live and viable) was the most promising, showing positive efficacy on the improvement of the skin differentiation process, on skin renewal and generally reinforcing the structure of the dermal compartment by increasing the collagen network. The combined administration of hyaluronic acid and the probiotic LPC-S01 DSM 26760 (P3) has therefore shown a synergistic effect in the reinforcement of the dermis structure, intervening on key factors of cell differentiation and renewal. This synergistic effect was not predictable since the administration of hyaluronic acid and the probiotic LPC-S01 DSM 26760 separately did not show a significant effect on skin differentiation.

### I.2. INACTIVATED bacterial strain - HOMEOSTATIC MODEL

### I.2.1. Products under analysis and Control.

- negative Control (NC): 0.9% NaCl saline solution;
- P1-i.: inactivated strain LPC-S01 DMS 26760 resuspended in saline solution (10⁹ cells/tissue), use concentration 2 g LPC-S01/ml;
- P2-i.: hyaluronic acid-based cream (as in Example-A (cream) reported above);
- P3-i.: inactivated strain LPC-S01 DSM 26760 resuspended in hyaluronic acid-based Cream (similar to P2-i.) (10⁹ cells/tissue), use concentration 2 g cells/ml.

To prepare P3-i., the content of a powder capsule (about 8×10⁹ CFU) of the freeze-dried inactivated strain LPC-S01 DSM 26760 was dissolved in a hyaluronic acid-based cream.

To prepare P1-i. (strain only), the content of a powder capsule (about 8×10⁹ CFU) of a freeze-dried inactivated strain LPC-S01 DSM 26760 was resuspended in a saline solution.

### I.2.2. Inactivation of the bacterial strain under analysis.

Starting material: viable bacterial cells of the strain LPC-S01 DSM 26760 in freeze-dried form with a viable cell count of 2 * 10¹¹ cells/g of powder.

The day before treatment, 2 g of powder were weighed and resuspended in 4 mL of saline solution to obtain a suspension of 10¹¹ bacterial cells/mL of saline solution. 30 µL of this suspension were used to verify the effective bacterial load by means of a count on the MRS agar.

On the day of treatment, the suspension of bacterial strains prepared in saline solution was heat-inactivated by incubating the bacterial suspension at 85°C for 1 hour. After this period, the bacteria were divided into 4 *eppendorf* test tubes (1 ml each, corresponding to 10¹¹ bacterial cells) and centrifuged. The pellet was then resuspended in:
- 1 ml of saline solution, obtaining P1-i.
- 1 ml of hyaluronic acid-based cream, obtaining P3-i.

### I.2.3. Methodology.

The products under analysis and in negative control (P1-i., P2-i., P3-i., NC: 15 µl) were applied directly on the surface of the T-Skin model tissues for 24 hours and 48 hours under homeostasis conditions. By applying 15 µL of suspensions of P1-i and P3-i., 10⁹ bacteria/tissue were applied.

The following parameters were analysed with respect to the untreated control (negative control, NC):
- Histomorphological analysis with H&E (Hematoxylin & Eosin) staining;
- Gene expression (by means of RT-qPCR) of the main biomarkers of skin defence (Human defensin β2 (DEFB4)), innate immune response (TLR2, TNFα), differentiation and epidermal renewal (TGMS-1, CCND1, TGF-β1).

The experiments were carried out in biological triplicate.

### I.2.4. Results.

### I.2.4.1. Results of the H&E histomorphological analysis at 24 hours and 48 hours

Three sections of vertical tissue (consisting of 3 replicates) are prepared on each histological slide; 5 microscopic acquisitions were performed on the selected section. For each biological replicate, the most representative acquisition of the selected vertical section is reported. The average thickness was calculated on 5 microscopic acquisitions.

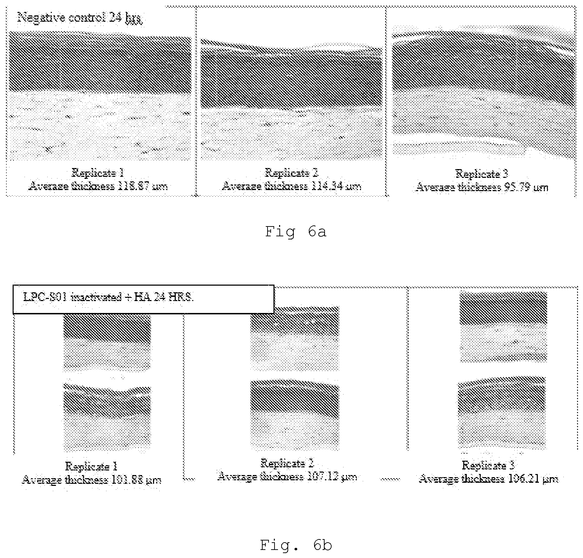

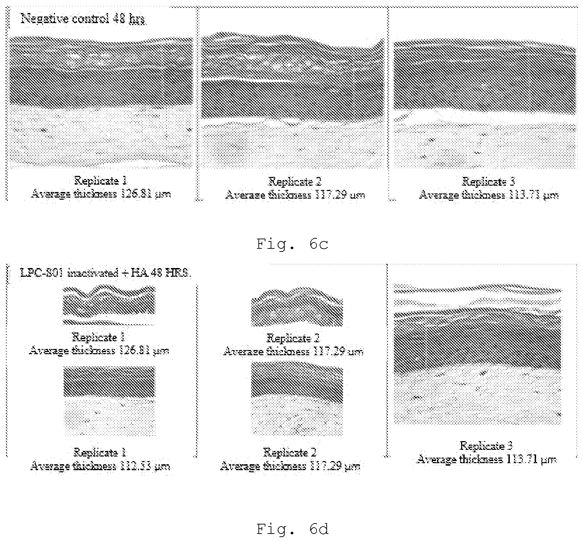

The results are reported below:
CN at 24hrs (Figure 6a):
   - Epidermis: fully viable and with regular SC *(stratum corneum)* lamellar structure;
   - Dermal-Epidermal Junction: the integrity of the structure is observed;
   - Fibres and collagen distribution: oriented and comprising many fibroblasts.
P1-i. at 24 hours (not shown in the figure):
   - Epidermis: differences are observed in the same replicate, probably linked to a non-homogeneous distribution; many cells are visible showing metabolic activation and proliferation; few pyknotic nuclei; the SC lamellar structure does not appear to be significantly modified;
P3-i. at 24 hours (Figure 6b):
   - Epidermis: differences are observed in the same replicate, probably linked to a non-homogeneous distribution; many cells showing metabolic activation and proliferation are visible; few pyknotic nuclei; the SC *(stratum corneum)* lamellar structure appears significantly modified;
   - Dermal-epidermal junction: the integrity of the structure is observed;
   - Fibres and collagen distribution: oriented dense, compact and including many fibroblasts.
NC at 48 hours (Figure 6c):
   - Epidermis: fully viable and with regular SC *(stratum corneum)* lamellar structure;
   - Dermal-epidermal junction: detachment and loss of integrity (intrinsic fragility of tissues) observed;
   - Fibres and collagen distribution: oriented and comprising many fibroblasts.
P1-i. at 48 hours (not shown in the figure):
   - Epidermis: a completely differentiated epidermis is observed; the tissue shows a modified SC - in terms of structure and thickness - due to greater proliferation and differentiation.
P3-i. at 48 hours (Figure 6d):
   - Epidermis: differences are observed in the same replicate, probably linked to a non-homogeneous distribution; many cells showing metabolic activation are visible and they proliferate; few pyknotic nuclei; the SC *(stratum corneum)* lamellar structure appears modified due to greater proliferation;
   - Dermal-epidermal junction: the integrity of the structure is observed;
   - Fibres and collagen distribution: oriented and comprising many fibroblasts; more dense and compact with respect to the negative control.

The data reported above demonstrate that the composition P3-i. (inactivated strain + HA) acts in shorter times and with greater efficacy than the P1-i composition (inactivated strain only), given that after applying to the P3-i tissue at 24 hours the lamellar structure of SC *(stratum corneum)* appears modified, while after applying P1-i. it is necessary to wait for 48 hours before observing a modification of the SC lamellar structure.

### I.2.4.2. Real time PCR results

### I.2.4.2.1. Real time PCR results at 24 hours

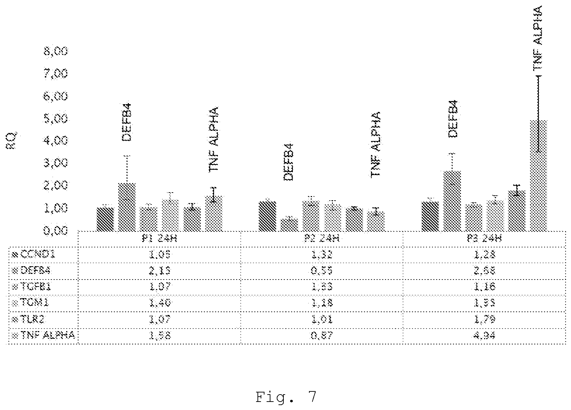

Figure 7 shows the results of gene expression (qRT-PCR) at 24 hours (RQ calculated with NC at 24 hours=1; RQ<0.5 de-regulation, RQ>2 upregulation)

The results of relative quantitation (RQ) gene expression (qRT-PCR) obtained in tissues treated with the compositions under analysis (P1-i., P2-i., P3-i.) at 24 hours are summarised in Figure 7 below. The results are expressed with respect to the negative control (NC at 24 hours = 1; RQ<0.5 de-regulation, RQ>2 upregulation).

As a general consideration, the non-fully homogeneous application of the compositions under analysis led to a high biological variability between the three replicates.

After 24 hours of treatment:
- The inactivated probiotic strain under analysis, alone (P1-i) or included in the composition comprising HA (P3-i), induced the upregulation of Human Defensin β2 (DEFB4). These data are consistent with the immunomodulating properties of the viable strain LPC-S01 DMS 26760 observed in section 1.1.3.1. and they indicate its ability to trigger skin defences even in a non-viable condition. Therefore, this immunomodulatory activity may be correlated with externally exposed elements in the bacterial cell wall.
- The composition comprising HA only (P2-i.) did not modulate Human defensin β2 (DEFB4): this result confirms that the upregulation of DEFB4 in P3-i. (strain+HA) is linked exclusively to the presence of the bacterium.
- the composition P3-i. (strain + HA) induced the upregulation of Human defensin β2 (DEFB4) at 24 hours in a higher (though not significant) manner as compared to P1-i. (strain only). A significant regulation of TNFα was quantified indicating the induction of an inflammatory response probably due to the immune recognition of bacteria by the surface of the skin. Since this effect is not observed in the tissue treated with P2-i. (HA only), this result can be linked to the high dose of strain LPC-S01 DMS 26760 included in composition P3-i.

### I.2.4.2.2. Real time PCR results at 48 hours

The induction of Human defensin β2 (DEFB4) by the inactivated bacterial strain under analysis (both in P1-i. and P3-i.) is confirmed, with values higher than the 24 hour time point (section I.2.4.2.1.), indicating a stable biological response and reinforcement of skin defences, resulting in an effective protection mechanism.

### I.2.5. Conclusions.

In the homeostatic (non-inflammatory) model, the composition according to the invention P3-i. (inactivated strain + HA), comprising the combination of an inactivated LPC-S01 DMS 26760 bacterial strain and hyaluronic acid, was well tolerated in the 3D skin model and was capable of stimulating the body's defences and cell differentiation processes as compared to the individual components and/or the negative control.

In particular, the composition of the invention P3-i. (inactivated strain + HA) efficacy levels are reached in shorter times as compared to the bacterial strain not in combinations with hyaluronic acid (P1-i.).

### II. INFLAMMATORY MODEL on Episkin T-Skin^{™} model

The capacity of the compositions according to the invention, comprising a bacterial strain LPC-S01 DMS 26760 (viable or inactivated) and hyaluronic acid, to reduce the damage caused by UV radiation was evaluated on a full 3D in vitro reconstructed skin model that reproduces the compartments of the dermis and epidermis (T-Skin^{™} model), thus allowing to study the changes of the extracellular matrix of the dermis and the differentiation of the viable layers (full thickness skin model).

Said evaluation was carried out according to two models, inflammatory model A (pre-treatment on non-damaged tissue) and inflammatory model B (pre- and post-treatment on damaged tissue), reported below.

### II.A. INFLAMMATORY MODEL A for VIABLE or INACTIVATED BACTERIAL STRAIN

### II.A.1. Products under analysis and Controls.

- P1: viable LPC-S01 DMS 26760 resuspended in saline solution,
- P2: hyaluronic acid-based solution (see I.1.1. Mask);
- P3: viable LPC-S01 DMS 26760 resuspended in a hyaluronic acid-based solution,
- P1-i.: inactivated LPC-S01 DMS 26760 and resuspended in saline solution,
- P2-i.: hyaluronic acid-based solution (see I.1.1. Mask);
- P3-i.: Inactivated LPC-S01 DMS 26760 resuspended in a hyaluronic acid-based solution,
- Positive control (PC): tissue treated with a 0.9% NaCl saline solution, abraded and exposed to UV radiation.
- Negative control (NC): tissue treated with 0,9% NaCl saline solution without exposure to UV radiation.

P1 (viable strain only) and P3 (viable strain + HA) were prepared by resuspending the content of a capsule of freeze-dried bacterial strain (CFU 8×10⁹) in 13 ml of saline solution comprising hyaluronic acid, respectively.

P1-i. (inactivated strain only) and P3-i. (inactivated strain + HA) were prepared by resuspending the contents of a freeze-dried bacterial strain capsule (CFU 8×10⁹) in a saline solution and incubated at 85°C for 1 hour. After this period the bacteria were centrifugated and the pellet was suspended in 13 ml of hyaluronic acid-based saline solution (or cream), respectively.

### II.A. Method of evaluating the reduction of UV damage

The reduction of UV damage of the probiotic strain LPC-S01 DSM 26760 (live and viable or inactivated) was evaluated on the "Full thickness skin" model, as described above. The study regarded the effect of the strain LPC-S01, hyaluronic acid and a composition comprising the strain LPC-S01 DSM 26760 + hyaluronic acid on the morphology of haematoxylin-eosin marked tissues and on the activation of the inflammasome in response to UV irradiation. The strain LPC-S01 DSM 26760, hyaluronic acid and the composition comprising the probiotic strain LPC-S01 DSM 26760 + hyaluronic acid were applied directly to the surface of the 3D skin model, incubated overnight and subsequently rinsed using saline solution to eliminate the excess product (pre-treatment step). The tissue was slightly abraded and then exposed to 1 MED (minimum erythemogenic dose) of UV to mimic normal solar exposure. Activation of inflammation was tested 4 and 24 hours after exposure to UV rays. A tissue treated with saline solution and exposed to UV rays was used as positive control. A tissue treated with a saline solution and not exposed to UV was used as negative control.

The evaluation of the efficacy of the compositions under analysis in reducing the damage caused by UV rays was analysed, according to methods known to the man skilled in the art, by means of:
- NFkB immunostaining method;
- histomorphological analysis with haematoxylin/eosin (H&E) staining; and
- IL-1β quantification method (only on viable strain).

### II.A.3. Results of UV damage reduction (Inflammatory model A)

### II.A.3.1. Results of NFkB immunostaining

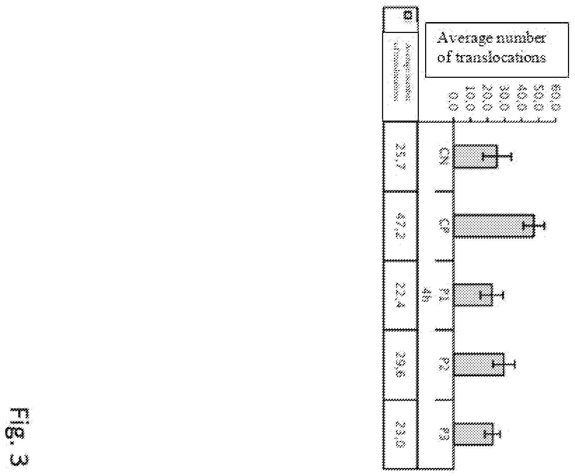

Figure 3 summarises the results of quantitation of NFkB translocation after 4 hours from exposure to UV rays (compositions under analysis comprising viable strains). 4 hours after irradiation, the positive control (PC) showed a high number of NFkB translocations, particularly in the suprabasal layer of the epidermis.

Treatments with LPC-S01 DSM 26760 (P1), with the strain LPC-S01 DSM 26760 and hyaluronic acid (P2) and with hyaluronic acid (P3) inhibit the nuclear translocation of NFkB significantly with respect to the positive control.

Treatment with probiotic LPC-S01 DSM 26760, LPC-S01 DSM 26760 + hyaluronic acid and hyaluronic acid has therefore shown a capacity to prevent activation of inflammation by inhibiting the translocation of NFkB in the nucleus of cells subjected to UV ray insult.

In addition, Table 2 shows the semi-quantitative data of the NFkB nuclear translocation determined for a pre-treatment time of 16 hours (long-term) with the compositions under analysis comprising the viable strains (P3) or the inactivated strains (P3-i), and evaluation of parameters 4 hours after exposure to UV rays, useful for assessing the effect of long-term treatment.

The biological relevance and reproducibility of said inflammasome model (UV exposure at 1 MED, minimal erythemal dose) was confirmed by an increase in the NFkB translocation in the cell nucleus in the irradiated samples (positive control) as compared to the non-irradiated samples (negative control).

The relative increase in NFkB translocation (percentage difference) in the study with composition P3-i with inactivated strains (+ 70.7%, w = 0.01) is comparable to that quantified in the study with composition P3 with viable strains (+83.7, w = 0.01).

**Table 2**

| | | NFkB translocation (Mean±st.dev.) | |
|---|---|---|---|
| Negative control | NC | 11.0±2.1 | 25.7±8.3 |
| Positive control | PC | 18.8±4.8 (+70.7% w=0.01) | 47.2±6.2 (+83.7 w=0.01) |
| Treatment | | 16h (ON) | 16h (ON) |
| Strain (viable) + HA | P3 | - | 29.6 ± 6.3 (-37.3%w=0.00) |
| Strain (inactive) + HA | P3-i | 16.0±3.2 (+14.8) | - |

Furthermore, at 24 hours P3 showed a reduction in the cytoplasmic content of NFkB as compared to the positive control (data not measured for P3-i).

### II.A.3.2. Histomorphological results with H&E staining.

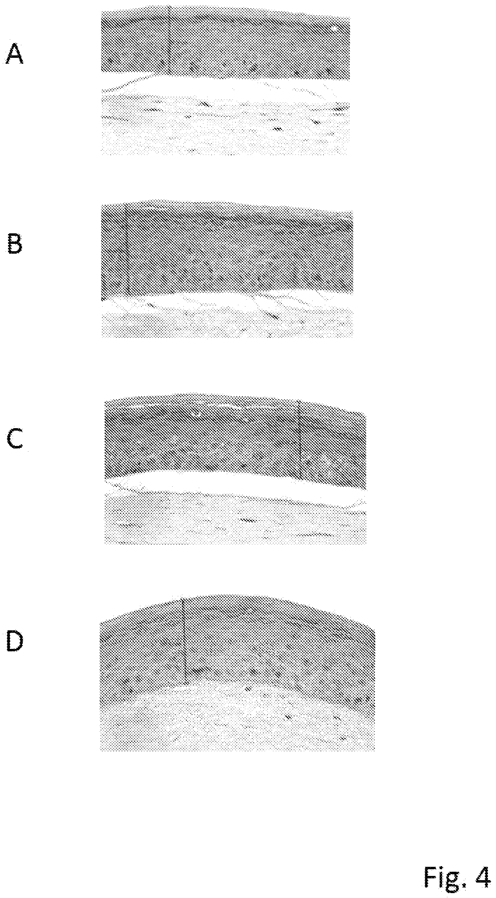

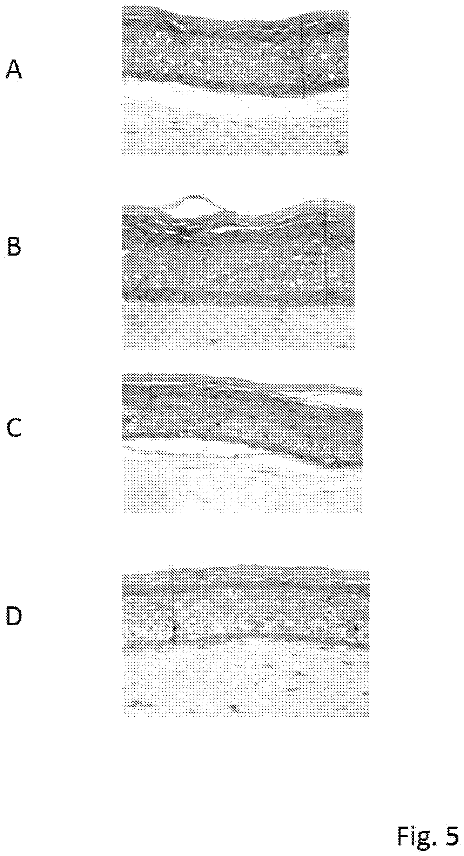

Figure 4 and 5 show the tissues treated with a saline solution (positive control) (A), with the probiotic LPC-S01 DSM 26760 (B), with hyaluronic acid (C) and with LPC-S01 DSM 26760 (viable) + hyaluronic acid (D) respectively 4 and 24 hours after the insult with UV rays (histomorphology with H&E staining).

As it can be seen, in both figures, the treatment with hyaluronic acid and the treatment with the probiotic LPC-S01 DSM 26760 is not capable of reducing the damage induced by UV rays. In particular, signs of UV-ray-induced sunburns are visible in the basal layer and in the spinous layer of the epidermis. Furthermore, the dermal-epidermal junction is damaged by UV rays and the epidermis does not adhere completely to the dermis, a sign of an altered skin structure (Figures 4A, 4B, 5A and 5B).

The treatment with hyaluronic acid + probiotic LPC-S01 DSM 26760 is capable of reducing the damage induced by UV after 4 and 24 hours from the induction of the damage. In particular, the structure of both the dermis and the epidermis is more compact as compared to the treatment with hyaluronic acid and with the probiotic LPC-S01 DSM 26760 administered individually. Furthermore, the structure of the dermal-epidermal junction is better maintained, favouring a better adherence of the epidermis to the dermis (Figures 4D and 5D).

This synergistic effect of the joint administration of hyaluronic acid and probiotic LPC-S01 DSM 26760 was not predictable given that neither hyaluronic acid alone nor the probiotic administered alone was capable of reducing the "release" of the epidermis from the dermis, and therefore, it could not maintain the physiological structure of the skin.

### II.A.3.3. II-1β quantification results

IL-1β was quantified at 4 hrs for the composition according to the invention P3, comprising the viable strain and hyaluronic acid, toward negative control and the positive control (Table 3). The results cannot be considered quantitative data given that the signal was below the kit detection limit (3.91 Pg / mL limit). The results are indicated to give a global trend.

**Table 3**

| | NC | PC | P3a |
|---|---|---|---|
| IL-1β (pg/ml) | 2.12 | 2.74 | 1.84 |

### II.A.4. Conclusions

An experimental model on T-skin^{™} (full-thickness skin) based on inflammatory pathways induced by UVA + UVB (1 MED dose) was used to evaluate the efficacy of the compositions according to the invention P3 and P3-i, comprising a bacterial strain LPC-S01 DMS 26760 (viable or inactivated, respectively) and hyaluronic acid, when applied before the induction of inflammasome stress (pre-treatment).

The compositions according to the invention P3 (viable strain + HA) and P3-i (inactivated strain + HA) showed good efficacy in reducing the translocation of NFkB in long-term pre-treatment (16 hours).

Furthermore, the composition according to the invention P3 (viable strain + HA) showed a good capacity to protect the structure of the dermal-epidermal junction from UV rays in the histomorphology study with H&E staining.

### II.B. INFLAMMATORY MODEL B for INACTIVATED STRAIN

An experimental model on T-skin^{™} (full-thickness skin) based on inflammatory pathways induced by UVA + UVB (1 MED dose) was used to evaluate the efficacy of the composition according to the invention P3-i., comprising an inactivated bacterial strain LPC-S01 DMS 26760 and hyaluronic acid, applied to damaged tissue before or after induction of inflammasome stress (pre-treatment or post-treatment with respect to UV irradiation).

The compositions under analysis (see II.B.1) were evaluated using a high concentration of inactivated bacterial strain under analysis (10⁷ or 10⁹ cells/tissue) with the aim of exploring their potential application and efficacy on the T-Skin inflammasome model according to 2 protocols:
B.I. PRE-TREATMENT PROTOCOL: T-skin abraded through a mechanical stress on the epidermal surface and pre-treated for 45 minutes or 4 hours with the compositions under examination, then subjected to UVA and UVB irradiation (1 MED). 4 hours after said irradiation (post-incubation), tissues were collected for analysis.
B.II. POST-TREATMENT PROTOCOL: T-skin abraded by mechanical stress on the epidermal surface and subjected to UVA and UVB irradiation (1 MED), then treated for 45 minutes or 4 hours with the compositions under examination and immediately collected for analysis.

The purpose of the study was to study the efficacy of the bacterial strain in analysis inactivated at high doses (alone or mixed with HA) in modulating the activation and translocation of NFkB in the nucleus.

### II.B.1. Compositions under analysis and Controls.

- P3-i.-10⁹: Inactivated LPC-S01 DMS 26760 (10⁹ cells/tissue) resuspended in a hyaluronic acid-based Cream (see I.2.1.), corresponding to 30% of the final composition;
- P3-i.-10⁷: inactivated LPC-S01 DMS 26760 (10⁷ cells on tissue) resuspended in a hyaluronic acid-based Cream (see I.2.1.), corresponding to 0.03% of the final composition;
- Positive control (PC): tissue treated with a 0.9% NaCl saline solution, abraded and exposed to UV radiation.
- Negative control (NC): tissue treated with 0,9% NaCl saline solution without exposure to UV radiation.

P3-i. (inactivated strain + HA) was prepared by resuspending the contents of a freeze-dried bacterial strain capsule (CFU 10⁹) in a saline solution and incubated at 85°C for 1 hour. After this period the bacteria were centrifugated and the pellet was suspended in 13 ml of hyaluronic acid-based cream.

### II.B.2. Study design

### II.B.2.1. Preparation of the compositions under analysis

The inactivated strain under analysis in freeze-dried form having a number of cells was 2 * 10¹¹ cells/g of powder was weighed and resuspended in the correct solvents as follows:
- 2 g in 4 ml of HA-based cream (see I.2.1.), obtaining P3-10⁹. 10⁹ bacteria/tissues were applied by applying 15 µL of suspensions.
- 0.02 g in 4 ml of HA-based cream (see I.2.1.), obtaining P3-10¹. 10⁷ bacteria/tissues were applied by applying 15 µL of suspensions.

### II.B.2.2. Induction and treatment of inflammasome T-skin

### II.B.2.2.1. Pre-treatment protocol

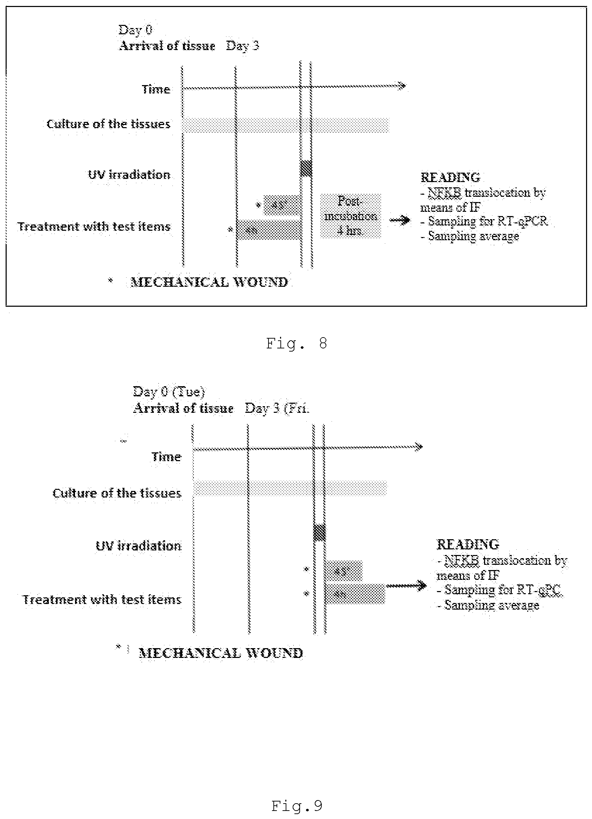

The experimental design is summarised in Figure 8: on the day of the experiment, the tissues were injured by a slight mechanical stress (Algerbrush n strokes = 2) and treated with 15 µL of analysis compositions (P3-i.) which were applied directly and uniformly to the T-Skin^{™} tissues and incubated for 45 minutes or 4 hours.

Subsequently, the tissues were irradiated with 1 MED (equivalent to 0.025 J/cm2), in PBS, using the Oriel 1KW solar simulator with xenon arc lamp and Irradiance WG320 [mW/cm 2] erythemal filter, (0.035 mW/cm2, according to calibration certificate n° 16121 issued by Opto.Cal GmbH). After induction of the inflammasome, tissues were post-incubated under homeostasis conditions for 4 hours and then fixed in formalin and incorporated in paraffin (FFPE) for NFκB immunostaining. Tissues were also collected for further RT-qPCR analysis. The carriers were collected and stored at -20⁰C.

### II.B.2.2.2. Post-treatment protocol

The experimental design is summarised in Figure 9: on the day of the experiment, the tissues were injured by a slight mechanical stress (Algerbrush n strokes = 2) and irradiated with 1 MED (equivalent to 0.025 J/cm²), in PBS. After the induction of the inflammasome, the tissues were treated with 15 µL of compositions under analysis and incubated for 45 minutes or 4 hours. Immediately after treatment, the tissues were collected and fixed in formalin for NFκB immunostaining. Tissues were also collected for further RT-qPCR analysis.

### II.B.3. Results of NFkB immunostaining

Table 4 below shows the results of the NFkB translocation (expressed as the total number of nuclei detected in 3 biological replicates) on the negative control with respect to the positive controls of each protocol. As reported in Table 4, induction of the inflammasome model was confirmed by an increase in the NFkB translocation of in the cell nucleus in the irradiated samples with respect to the to the negative control.

However, in this T-skin batch an earlier NFkB activation was observed after irradiation, although translocation was observed at each time point. In particular, the highest induction of NFkB is observed 45 minutes after irradiation.

The protocols adopted are based on reading after UV irradiation in the post-treatment model and reading after post-incubation in the pre-treatment model, given that the objective is to assess the efficacy of the products on recovery from the acute inflammatory process.

**Table 4**

| | |
|---|---|
| Negative control | 0 |
| Positive control 45 min pre-treatment + 4 hours after UV incubation | 7 |
| Positive control 4 hours pre-treatment + 4 hours UV incubation | 5 |
| Positive control 45 minutes after UV irradiation | 9 |
| Positive control 4 hours after UV irradiation | 4 |

### A) 45-minute pre-treatment

In Table 5, the semi-quantitative analysis of the NFκB nuclear translocation of (expressed as the total number of nuclei detected in all biological replicates) is presented for the 45-minute pre-treatment + 4 hours of post-UV irradiation incubation.

**Table 5. composition according to the invention**

| | | |
|---|---|---|
| Negative control | NC | 0 |
| Positive control | PC | 7 |
| inactivated strain 10⁹ + HA* | P3-i.(10⁹) | 1 |
| inactivated strain 10⁷ + HA * | P3-i.(10⁷) | 5 |

### B) 4-hour pre-treatment

In Table 6, the semi-quantitative analysis of the NFκB nuclear translocation (expressed as the total number of nuclei detected in all biological replicates) is presented for the 4-hour pre-treatment + 4 hours of post-UV irradiation incubation.

**Table 6. composition according to the invention**

| | | |
|---|---|---|
| Negative control | NC | 0 |
| Positive control | PC | 5 |
| inactivated strain 10⁹ + HA* | P3-i.(10⁹) | 1 |
| inactivated strain 10⁷ + HA * | P3-i.(10⁷) | 2 |

Analysis of the results of Table 5 and 6:
- When applied as a pre-treatment, the composition P3-i., comprising the inactivated strain LPC-S01 DMS 26760 and hyaluronic acid, was capable of reducing the NFkB translocation in the nucleus, indicating a preventive efficacy in protecting the skin from the inflammatory stress induced by UV rays.
- At a concentration of 10⁹ of strain, in the 45-minute pre-treatment model, the composition P3-i. (inactivated strain+HA) was capable of highly reducing the NFkB translocation in the nucleus indicating a synergistic and/or highly effective effect between the inactivated LPC-S01 DMS 26760 strain and the hyaluronic acid.
- The tissue response to the pre-treatment of the compositions P3-i.(10⁹) and P3-i.(10⁷) at 2 different concentrations of inactivated LPC-S01 DMS 26760 suggests a dose-response mechanism: fewer NFkB positive nuclei were detected at increasing strain concentrations in the composition.

### C) 45-minute post-treatment

In Table 7, the semi-quantitative analysis of the NFκB nuclear translocation (expressed as the total number of nuclei detected in all biological replicates) is presented for the 45-minute pre-treatment after UV irradiation.

**Table 7. composition according to the invention**

| | | |
|---|---|---|
| Negative control | NC | 0 |
| Positive control | PC | 9 |
| inactivated strain 10⁹ + HA* | P3-i.(10⁹) | 3 |
| inactivated strain 10⁷ + HA * | P3-i.(10⁷) | 0 |

### D) 4-hour post-treatment

In Table 8, the semi-quantitative analysis of the NFκB nuclear translocation (expressed as the total number of nuclei detected in all biological replicates) is presented for the 4-hour pre-treatment after UV irradiation.

**Table 8. composition according to the invention**

| | | |
|---|---|---|
| Negative control | NC | 0 |
| Positive control | PC | 4 |
| inactivated strain 10⁹ + HA* | P3-i.(10⁹) | 1 |
| inactivated strain 10⁷ + HA * | P3-i.(10⁷) | 9 |

- The composition P3 (inactivated strain + HA) has demonstrated a rapid and effective recovery of the basal levels of NFKB, particularly in the short term (when the inflammatory response is at its maximum level), suggesting an improvement synergy/effect in the combination of inactivated strain LPC-S01 DMS 26760 + hyaluronic acid in restoring the homeostasis of inflamed tissues.

### II.B.4. Conclusion

These results confirm that the composition P3-i comprising the inactivated LPC-S01 DMS 26760 + hyaluronic acid combination is effective both in homeostatic conditions (see section I.2.) and in conditions of inflammation of the skin (e.g. caused by UV radiation), particularly in the acute phase of inflammation given that it is particularly effective in the short term of the development of inflammation.

### EXPERIMENTAL PART (2)

### Evaluation of the adhesion of Cutibacterium acnes DSM 1897 to the 3D "Full Thickness Skin" model in the presence of a composition according to the invention (strain Lactobacillus paracasei LPC-S01 DSM 26760 and hyaluronic acid)

### 1. Objective of the study.

The objective of the study was to evaluate the ability of the probiotic strain *L. paracasei* LPC-S01 DSM 26760, alone and/or in combination with hyaluronic acid (HA), to combat *in-vitro* adhesion of *C. acnes* to a "full thickness skin" model.

*Cutibacterium acnes* (in short *C. acne,* also known as *Propionibacterium acnes or P. acne* (Douglas et Günter, 1946)) is a slow-growing Gram-positive anaerobic bacterium linked to certain skin diseases, such as acne; it may also be the cause of blepharitis and endophthalmitis.

In order to assess various possible infection situations, a competition model and an exclusion model were implemented, based on the adaptation of the method described by Coman et al. in 2015.

### 2. Experimental design.

The strain *C. acnes* DSM 1897 was used to simulate infection in a 3D "Full Thickness Skin" model, purchased, for a total of 30 inserts, from Phenion (Henkel).

The tests were carried out considering different treatment conditions, listed below:
1) no treatment, to evaluate the effective adhesive capacity of *C. acnes* DSM1897 in the 2 exclusion and competition models;
2) preventive or concomitant treatment with *L. paracasei* LPC-S01 DSM 26760 for 24 hours;
3) preventive or concomitant treatment with 0.5% hyaluronic acid for 24 hours (Sigma-Aldrich 41897);
4) preventive or concomitant treatment with a homogeneous mixture of hyaluronic acid and *L. paracasei* LPC-S01 DSM 26760 for 24 hours;
5) preventive or concomitant treatment with benzoyl peroxide for 24 hours (Benzac 10%, positive control).

A suspension of the strain L. paracasei LPC-S01 DSM 26760 was prepared and 50 µl of the suspension were placed at contact with the surface of the insert.

A 0.5% hyaluronic acid suspension was prepared and 50 µl of the suspension were placed at contact with the inserts.

Furthermore, 50 µl of strain *L. paracasei* LPC-S01 DSM 26760 were mixed with 0.25 mg of hyaluronic acid to obtain a combined preparation of probiotic + hyaluronic acid, at the same concentration of hyaluronic acid (0.5%) and at the same initial load used for tests with the individual substances.

50 µl of Benzac gel 10% (benzoyl peroxide) were placed at contact with the inserts as a positive control.

All 5 conditions listed above were tested in duplicate and the 10 inserts were incubated for 24 hours at 37°C in the presence of CO2.

### 2.a. Execution of the exclusion test

The exclusion test provided for the pre-treatment of the inserts with the probiotic strain (or with hyaluronic acid or with the mixture of the two), the subsequent infection with the pathogen and the subsequent verification of the possible reduction of the % adhesion of the pathogen to the insert with respect to the ideal condition of infection (*in-vitro* model of preventive probiotic treatment).

### 2.b. Execution of the competition test

The competition test provided for the concomitant treatment of the inserts with the probiotic strain (or with hyaluronic acid or with the mixture of the two) and with the pathogen and the subsequent verification of the possible reduction in the % adhesion of the pathogen to the insert with respect to the ideal condition of infection (in-vitro model of probiotic treatment during the course of infection).

### 3. Results

### 3.1 Exclusion test

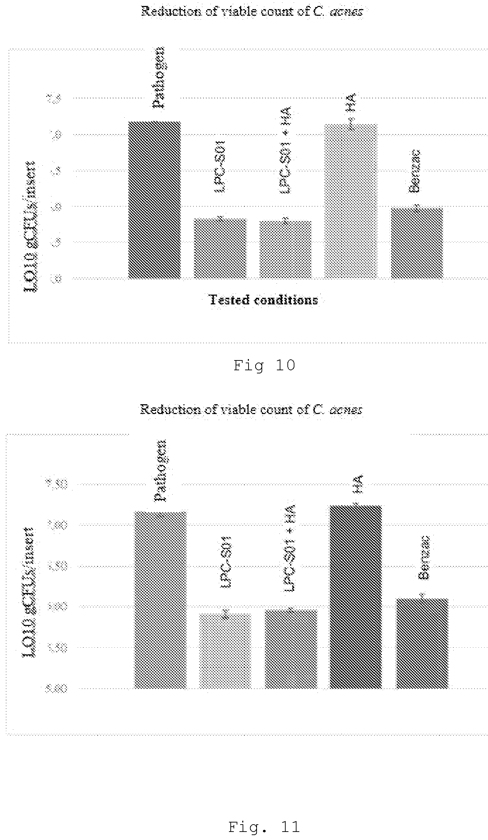

Figure 10 shows the reduction in viability of the pathogen *C. acnes* DMS1897 expressed in Log10 CFUs, while Table 9 shows the same situation as a percentage reduction (%) of the pathogen viability under the various tested conditions.

**Table 9**

| Tested condition | % reduction of the pathogen |
|---|---|
| LPC-S01 | 19.1 |
| LPC-S01 + hyaluronic acid | 19.5 |
| Hyaluronic acid | 0.8 |
| Benzac | 17.0 |

As can be seen from the reported results, the treatments carried out with Benzac 10%, LPC-S01 DSM 26760 and the combination of LPC-S01 DSM 26760 in the presence of 0.5% hyaluronic acid reduce the viability count of *C. acnes* DSM1897 by about 1.0-1.4 Log10, corresponding to approximately 20% reduction in pathogen viability. The treatment with 0.5% hyaluronic acid alone does not seem to be capable of reducing the viability of the pathogen to any extent.

### 3.2. Competition test

Figure 11 and Table 10 show the charts of the viable counts, expressed as logarithm reduction logCFUs of *C. acnes* DSM1897 on insert, of the mean obtained for the duplicate of each tested condition and as percentage reduction obtained in the competition test.

**Table 10**

| Tested condition | % reduction of the pathogen |
|---|---|
| LPC-S01 | 18.0 |
| LPC-S01 + hyaluronic acid | 17.3 |
| Hyaluronic acid | 0.0 |
| Benzac | 15.3 |

Based on the data presented, it is confirmed that the treatments carried out with Benzac 10%, LPC-S01 DSM 26760 and the combination of LPC-S01 DSM 26760 in the presence of 0.5% hyaluronic acid reduce the viability count of *C. acnes* DSM1897 by 1.0-1.3 Log10 CFUs. As with the exclusion test, the treatment with 0.5% hyaluronic acid alone does not seem to be capable of reducing the viability of the pathogen.

### 4. Conclusion

All *in-vitro* tests performed demonstrated the efficacy of the Benzac 10% positive control in the containment of infection by *C. acnes,* with % reduction in the viability of the pathogen population from 15% to 23%.

Exclusion and competition tests have shown that the treatments carried out with the combination of LPC-S01 + 0.5% hyaluronic acid (compositions according to the invention) reduce the infection by *C. acnes* DSM1897 by about 18-19%.

## Claims

1. A composition comprising:
- a mixture M comprising or, alternatively, consisting of:
(I) a bacterial strain belonging to the species *Lactobacillus paracasei* identified as *Lactobacillus paracasei* LPC-S01 and deposited at *Deutsche Sammlung von Mikroorganismen und Zellkulturen GmbH* (DSMZ) under the accession number DSM 26760, and
(II) hyaluronic acid or a salt thereof;
and, optionally, said composition comprises
- at least one acceptable pharmaceutical or cosmetic or food grade additive and/or excipient.

2. The composition according to claim 1, wherein said bacterial strain *Lactobacillus paracasei* LPC-S01 DSM 26760 is a viable bacterial strain.

3. The composition according to claim 1, wherein said bacterial strain *Lactobacillus paracasei* LPC-S01 DSM 26760 is a thermally inactivated or tyndallized or sonicated or gamma irradiated bacterial strain; preferably thermally inactivated.

4. The composition according to any one of claims 1-3, wherein said composition is formulated for topical skin use, preferably a cream or a mask.

5. The composition according to any one of claims 1-3, wherein said composition is formulated for oral use, preferably a suspension or a preparation for the formation of an extemporaneous suspension.

6. The composition according to any one of claims 1-5 for use as medicament.

7. The composition according to any one of claims 1-5 for use in a method for preventive and/or curative treatment of an inflammation and/or infection of the skin, or an associated disease or symptom, wherein said treatment comprises the administration of the composition to a subject in need.

8. The composition for use according to claim 7, wherein said inflammation and/or infection of the skin is induced by UV rays.

9. The composition for use according to claim 7, wherein said inflammation or infection of the skin is induced by pathogenic agents, preferably by *Cutibacterium acnes* or *Propionibacterium acnes.*

10. The composition according to any one of claims 1-5 for use in a method for preventive and/or curative treatment of a damage caused or induced to the skin by:
- UV radiation; and/or
- adverse weather conditions for the skin, preferably sunrays, cold or wind; and/or
- life conditions detrimental to the skin, preferably pollution, smoking or consumption of alcohol; and/or of an associated disease or symptom, wherein said treatment comprises the administration of the composition to a subject in need.

11. The composition for use according to any one of claims 7-10, wherein said inflammation and/or infection of the skin or said damage induced to the skin, or said associated diseases or symptoms, are selected from among the group comprising or, alternatively, consisting of:
acute or chronic skin inflammation or infection of the skin, bacterial or viral or fungal infection of the skin, abscess, aposteme, empyema, phlegmon, whitlow, furuncle, carbuncle, hidradenitis suppurativa, erysipelas, psoriasis, atopic dermatitis, acne, acute or chronic dermatosis, rosacea, couperosa, erythema, skin redness, burn, sunburn, reactivation of oral herpes, pressure ulcers, ulcers, rhagades, fistula, sore, wound, bruise, abrasion, ecchymosis, haematoma, excoriation, keratosis, hyperkeratosis, keloid.

12. Topical cosmetic use of the composition according to any one of claims 1-4, wherein said use is:
- for the maintenance of homeostasis of the skin, and/or
- as an anti-aging skin agent.

13. Cosmetic use according to claim 12 wherein said composition is for topical cosmetic use in the treatment of: wrinkles, loss of skin elasticity or solar elastosis, dry skin, rough skin, photo-aging, skin redness, presence of dilated capillaries on cheeks, nose and/or ears, sunspots, abnormal or uneven pigmentation or hyperpigmentation of skin.

## Patentansprüche

1. Zusammensetzung, umfassend:
- eine Mischung M, die Folgendes umfasst oder alternativ daraus besteht:
(I) einem Bakterienstamm, gehörend der Art *Lactobacillus paracasei,* identifiziert als *Lactobacillus paracasei* LPC-S01 und hinterlegt bei der *Deutschen Sammlung von Mikroorganismen und Zellkulturen GmbH* (DSMZ) unter der Hinterlegungsnummer DSM 26760, und
(II) Hyaluronsäure oder ein Salz davon;
und gegebenenfalls umfasst die Zusammensetzung
- mindestens einen annehmbaren pharmazeutischen oder kosmetischen oder lebensmittelechten Zusatzstoff und/oder Hilfsstoff.

2. Zusammensetzung nach Anspruch 1, wobei der Bakterienstamm *Lactobacillus paracasei* LPC-S01 DSM 26760 ein lebensfähiger Bakterienstamm ist.

3. Zusammensetzung nach Anspruch 1, wobei der Bakterienstamm *Lactobacillus paracasei* LPC-S01 DSM 26760 ein thermisch inaktivierter oder tyndallisierter oder beschallter oder gammabestrahlter Bakterienstamm ist; vorzugsweise thermisch inaktiviert.

4. Zusammensetzung nach einem der Ansprüche 1-3, wobei die Zusammensetzung für die topische Hautanwendung formuliert ist, vorzugsweise eine Creme oder eine Maske.

5. Zusammensetzung nach einem der Ansprüche 1-3, wobei die Zusammensetzung zur oralen Verwendung formuliert ist, vorzugsweise eine Suspension oder ein Präparat zur Bildung einer extemporären Suspension.

6. Zusammensetzung nach einem der Ansprüche 1-5 zur Verwendung als Medikament.

7. Zusammensetzung nach einem der Ansprüche 1-5 zur Verwendung in einem Verfahren zur vorbeugenden und/oder heilenden Behandlung einer Entzündung und/oder Infektion der Haut oder einer damit assoziierten Krankheit oder eines damit assoziierten Symptoms, wobei die Behandlung die Verabreichung der Zusammensetzung an ein bedürftiges Subjekt umfasst.

8. Zusammensetzung zur Verwendung nach Anspruch 7, wobei die Entzündung und/oder Infektion der Haut durch UV-Strahlen induziert wird.

9. Zusammensetzung zur Verwendung nach Anspruch 7, wobei die Entzündung oder Infektion der Haut durch pathogene Mittel, vorzugsweise durch *Cutibacterium acnes* oder *Propionibacterium* acnes, induziert wird.

10. Zusammensetzung nach einem der Ansprüche 1-5 zur Verwendung in einem Verfahren zur vorbeugenden und/oder heilenden Behandlung einer Schädigung, die an der Haut verursacht oder induziert wird durch:
- UV-Strahlung und/oder
- widrige Witterungsbedingungen für die Haut, vorzugsweise Sonnenstrahlen, Kälte oder Wind; und/oder
- Lebensbedingungen, die der Haut schaden, vorzugsweise Verschmutzung, Rauchen oder Alkoholkonsum;
und/oder einer assoziierten Krankheit oder eines Symptoms, wobei die Behandlung die Verabreichung der Zusammensetzung an ein bedürftiges Subjekt umfasst.

11. Zusammensetzung zur Verwendung nach einem der Ansprüche 7-10, wobei die Entzündung und/oder Infektion der Haut oder die an der Haut induzierte Schädigung oder die assoziierten Krankheiten oder Symptome aus der Gruppe ausgewählt sind, die Folgendes umfasst oder alternativ daraus besteht:
akute oder chronische Hautentzündung oder Infektion der Haut, bakterielle oder virale oder Pilzinfektion der Haut, Abszess, Apostem, Empyem, Phlegmone, Whitlow, Furunkel, Karbunkel, Hidradenitis suppurativa, Erysipel, Psoriasis, atopische Dermatitis, Akne, akute oder chronische Dermatose, Rosacea, Couperosa, Erythem, Hautrötung, Verbrennung, Sonnenbrand, Reaktivierung von oralen Herpes, Druckgeschwüren, Geschwüren, Rhagaden, Fisteln, wunde Stelle, Wunde, Blauer Fleck, Abrieb, Ekchymose, Hämatom, Exkoriation, Keratose, Hyperkeratose, Keloid.

12. Topische kosmetische Verwendung der Zusammensetzung nach einem der Ansprüche 1-4, wobei die Verwendung ist:
- zur Aufrechterhaltung der Homöostase der Haut und/oder
- als Anti-Aging-Hautmittel.

13. Kosmetische Verwendung nach Anspruch 12, wobei die Zusammensetzung zur topischen kosmetischen Verwendung bei der Behandlung von: Falten, Verlust der Hautelastizität oder Sonnenelastose, trockener Haut, rauer Haut, Lichtalterung, Hautrötung, Anwesenheit von dilatierten Kapillaren auf Wangen, Nase und/oder Ohren, Sonnenflecken, abnormaler oder ungleichmäßiger Pigmentierung oder Hyperpigmentierung der Haut ist.

## Revendications

1. Composition, comprenant :
- un mélange M comprenant ou, alternativement, constitué :
(I) d'une souche bactérienne appartenant à l'espèce *Lactobacillus paracasei* identifiée comme *Lactobacillus paracasei* LPC-S01 et déposée à *Deutsche Sammlung von Mikroorganismen und Zellkulturen GmbH* (DSMZ) sous le numéro d'accès DSM 26760, et
(II) de l'acide hyaluronique ou un sel de celui-ci ;
et, éventuellement, ladite composition comprend
- au moins un additif et/ou excipient acceptable de qualité pharmaceutique ou cosmétique ou de qualité alimentaire.

2. Composition selon la revendication 1, dans laquelle ladite souche bactérienne *Lactobacillus paracasei* LPC-S01 DSM 26760 est une souche bactérienne viable.

3. Composition selon la revendication 1, dans laquelle ladite souche bactérienne Lactobacillus *paracasei* LPC-S01 DSM 26760 est une souche bactérienne thermiquement inactivée ou tyndallisée ou soniquée ou irradiée aux rayons gamma ; de préférence thermiquement inactivée.

4. Composition selon l'une quelconque des revendications 1-3, dans laquelle ladite composition est formulée pour une utilisation topique sur la peau, de préférence une crème ou un masque.

5. Composition selon l'une quelconque des revendications 1-3, dans laquelle ladite composition est formulée pour une utilisation orale, de préférence une suspension ou une préparation pour la formation d'une suspension magistrale.

6. Composition selon l'une quelconque des revendications 1-5 à utiliser en tant que médicament.

7. Composition selon l'une quelconque des revendications 1-5 à utiliser dans une méthode de traitement préventif et/ou curatif d'une inflammation et/ou infection de la peau, ou d'une maladie ou symptôme associé, dans laquelle ledit traitement comprend l'administration de la composition à un sujet qui en a besoin.

8. Composition à utiliser selon la revendication 7, dans laquelle ladite inflammation et/ou infection de la peau est induite par les rayons UV.

9. Composition à utiliser selon la revendication 7, dans laquelle ladite inflammation ou infection de la peau est induite par des agents pathogènes, de préférence par *Cutibacterium acnes* ou *Propionibacterium acnes.*

10. Composition selon l'une quelconque des revendications 1-5 à utiliser dans une méthode de traitement préventif et/ou curatif d'un dommage causé ou induit sur la peau par :
- le rayonnement UV ; et/ou
- des conditions climatiques défavorables à la peau, notamment les rayons du soleil, le froid ou le vent ; et/ou
- des conditions de vie préjudiciables à la peau, notamment la pollution, le tabagisme ou la consommation d'alcool ;
et/ou d'une maladie ou d'un symptôme associé, dans lequel ledit traitement comprend l'administration de la composition à un sujet qui en a besoin.

11. Composition à utiliser selon l'une quelconque des revendications 7-10, dans laquelle ladite inflammation et/ou infection de la peau ou ledit dommage induit à la peau, ou lesdites maladies ou symptômes associés, sont choisis dans le groupe comprenant ou, alternativement, consistant en : l'inflammation ou l'infection aiguë ou chronique de la peau, l'infection bactérienne ou virale ou fongique de la peau, l'abcès, l'apostème, l'empyème, le phlegmon, le panaris, le furoncle, l'anthrax, l'hidrosadénite suppurée, l'érysipèle, le psoriasis, la dermatite atopique, l'acné, la dermatose aiguë ou chronique, la rosacée, la couperose, l'érythème, les rougeurs de la peau, les brûlures, les coups de soleil, la réactivation de l'herpès oral, les escarres, les ulcères, les rhagades, les fistules, les plaies, les blessures, les contusions, les abrasions, les ecchymoses, les hématomes, les excoriations, la kératose, l'hyperkératose, la chéloïde.

12. Utilisation cosmétique topique de la composition selon l'une quelconque des revendications 1-4, dans laquelle ladite utilisation sert :
- à maintenir l'homéostasie de la peau, et/ou
- comme agent anti-âge pour la peau.

13. Utilisation cosmétique selon la revendication 12, dans laquelle ladite composition est destinée à une utilisation cosmétique topique dans le traitement : des rides, de la perte d'élasticité de la peau ou de l'élastose solaire, de la peau sèche, de la peau rugueuse, du photovieillissement, des rougeurs de la peau, de la présence de capillaires dilatés sur les joues, le nez et/ou les oreilles, des taches solaires, de la pigmentation anormale ou irrégulière ou hyperpigmentation de la peau.
